(19) Europäisches Patentamt
European Patent Office
Office européen des brevets

(11) EP 2 563 930 B1

(12) **EUROPEAN PATENT SPECIFICATION**

(45) Date of publication and mention of the grant of the patent:
**25.11.2015 Bulletin 2015/48**

(21) Application number: **11774490.4**

(22) Date of filing: **02.05.2011**

(51) Int Cl.:
***C12Q 1/04*** *(2006.01)* ***C12Q 1/68*** *(2006.01)*
***C12R 1/145*** *(2006.01)*

(86) International application number:
**PCT/FI2011/050399**

(87) International publication number:
**WO 2011/135194 (03.11.2011 Gazette 2011/44)**

(54) **METHOD FOR DIAGNOSING RISK OF TYPE 1 DIABETES**

VERFAHREN ZUR DIAGNOSE DES RISIKOS VON DIABETES VOM TYP 1

PROCÉDÉ POUR DIAGNOSTIQUER UN RISQUE DE DIABÈTE DE TYPE 1

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.05.2010 US 330523 P**
**30.04.2010 FI 20105478**

(43) Date of publication of application:
**06.03.2013 Bulletin 2013/10**

(73) Proprietor: **Teknologian Tutkimuskeskus VTT OY**
**02150 Espoo (FI)**

(72) Inventors:
- **ORESIC, Matej**
  **FI-02044 Vtt (FI)**
- **SAARELA, Maria**
  **FI-02044 Vtt (FI)**
- **MAUKONEN, Johanna**
  **FI-02044 Vtt (FI)**
- **SYSI-AHO, Marko**
  **FI-02044 Vtt (FI)**

(74) Representative: **Seppo Laine Oy**
**Itämerenkatu 3 B**
**00180 Helsinki (FI)**

(56) References cited:
**JP-A- 2007 020 423 US-A1- 2007 225 480**

- **MARKO SYSI-AHO ET AL: "Metabolic Regulation in Progression to Autoimmune Diabetes", PLOS COMPUTATIONAL BIOLOGY, vol. 7, no. 10, 27 October 2011 (2011-10-27), page e1002257, XP055070905, ISSN: 1553-734X, DOI: 10.1371/journal.pcbi.1002257**
- **ROESCH, L.F.W. ET AL.: 'Culture-independent identification of gut bacteria correlated with the onset of diabetes in a rat model' THE ISME JOURNAL, [Online] vol. 3, no. 5, May 2009, pages 536 - 548, XP008161976 Retrieved from the Internet: <URL:http://www.nature.com/ismej/journal/v3 /n5/suppinfo/ismej20095s1.html> [retrieved on 2011-08-25]**
- **WEN, L. ET AL.: 'Innate immunity and intestinal microbiota in the development of Type 1 diabetes' NATURE vol. 455, no. 7216, October 2008, pages 1109 - 1113, XP008161978**
- **CALCINARO, F. ET AL.: 'Oral probiotic administration induces interleukin-10 0 production and prevents spontaneous autoimmune diabetes in the non-obese diabetic mouse' DIABETOLOGIA vol. 48, no. 8, August 2005, pages 1565 - 1575, XP019322640**
- **MATSUKI, T. ET AL.: 'Use of 16S rRNA gene-targeted group-specific primers for real-time PCR analysis of predominant bacteria in human feces' APPLIED AND ENVIRONMENTAL MICROBIOLOGY vol. 70, no. 12, December 2004, pages 7220 - 7228, XP008161970**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- SHEN, J. ET AL.: 'Molecular profiling of the Clostridium leptum subgroup in human fecal microflora by PCR-denaturing gradient gel electrophoresis and clone library analysis' APPLIED AND ENVIRONMENTAL MICROBIOLOGY vol. 72, no. 8, August 2006, pages 5232 - 5238, XP055005238
- LARSEN, N. ET AL.: 'Gut microbiota in human adults with type 2 diabetes differs from non-diabetic adults' PLOS ONE, [Online] vol. 5, no. 2, February 2010, page E9085, XP055004750 Retrieved from the Internet: <URL:http: //www.plosone.org/article/info:do i/ 10.1371/journal.pone.0009085> [retrieved on 2011-08-19]
- WU, X. ET AL.: 'Molecular characterisation of the faecal microbiota in patients with type II diabetes' CURRENT MICROBIOLOGY vol. 61, no. 1, June 2010, pages 69 - 78, XP019843446

**Description**

## FIELD OF THE INVENTION

This invention relates to a novel method for diagnosing an increased risk of type 1 diabetes in an individual. Furthermore, this invention relates to a method for preventing onset of type 1 diabetes in an individual.

## BACKGROUND OF THE INVENTION

The publications and other materials used herein to illuminate the background of the invention, and in particular, cases to provide additional details respecting the practice, are incorporated by reference.

Type 1 diabetes (T1D) is an autoimmune disease that results from the selective destruction of insulin-producing β-cells in pancreatic islets. The diagnosis of T1D is commonly preceded by a long prodromal period which includes seroconversion to islet autoantibody positivity[1] and subtle metabolic disturbances[2] . The incidence of T1D among children and adolescents has increased markedly in the Western countries during the recent decades[3] and is presently increasing at a faster rate than ever before[4,5]. This suggests an important role of environment and gene-environment interactions in T1D pathogenesis.

Metabolome is sensitive to both genetic and early environmental factors influencing later susceptibility to chronic diseases[6]. Recent evidence from serum metabolomics suggests that metabolic disturbances already precede β-cell autoimmunity markers in children who subsequently progress to T1D[2]. However, the environmental causes and tissue-specific mechanisms leading to these disturbances are unknown. Given its relatively low disease incidence in the general population and even among subjects at genetic risk[1], studies on early phenomena of T1D pathogenesis in humans are a huge undertaking as they require long and frequent follow-up of large numbers of subjects[2,7,8] to be able to go "back to the origins" of the disease once a sufficient number of subjects in the follow-up have progressed to overt disease.

The non-obese diabetic (NOD) mouse is a well characterized model of autoimmune disease[9] which has been widely used in studies of T1D. It is clear that the NOD experimental model does not completely mimic the immune system and T1D pathogenesis in man[10]. In fact, only a fraction of NOD mice progress to disease, with the incidence of spontaneous diabetes being 60%-80% in females and 20%-30% in males[9]. There is thus a stochastic component to T1D pathogenesis in NOD mice, believed to be due to random generation of islet-specific T cells[11]. The disease incidence does seem to depend on the environment and there is evidence indicating that its incidence is highest in relatively germ-free environment[12] and that gut microbiota may affect its incidence *via* the modulation of the host innate immune system[13].

WO 2008/031917 teaches using biofluid metabolite profile as a tool for early prediction of type 1 diabetes risk. Use of diabetes associated autoantibodies in connection of decreased metabolite level has been proposed for improving the accuracy. A nutritional intervention, an antioxidant therapy, or a stimulation of the biochemical synthesis of choline plasmalogens are proposed to be used for prevention of onset on the disease.

LUIZ FW ROESCH ET AL, THE I S M E JOURNAL, vol. 3, no. 5, 1 May 2009, pages 536-548, teaches identification of bacteria (in particular Lactobacillus and Bacteroides strais) correlated with the onset of diabetes in a rat model.

However, ther is still a need for improved methods for early identification of the individuals being susceptible to type 1 diabetes having increased risk of type 1 diabetes. There is also a need for effectively prevent onset of type 1 diabetes.

## SUMMARY OF THE INVENTION

Aim of the present invention is to provide a method according to appended claim 1 for diagnosing an increased risk of type 1 diabetes in an individual comprising the steps of

(a) providing a fecal sample;
(b) analyzing diversity of the *Clostridium leptum* group of said sample
(c) comparing the result to control samples obtained from healthy individuals;
(d) determining the difference between the patient samples and control samples,

wherein diminished diversity of *Clostridium leptum* group bacteria indicates an increased risk of type 1 diabetes.

The present invention provides a considerable advantage of enabling the individuals having a risk of type 1 diabetes being diagnosed at an early stage before or near the onset of islet autoimmunity. Especially individuals with increased risk should be diagnosed as early as possible.

Gut microbiota sample is obtained from lower gastrointestinal tract, preferably it is e.g. feces sample.

Once diagnosed at an early stage as belonging to the risk group, the onset of type 1 diabetes in the individual can be prevented by normalizing the diversity of the gut microbiota. Specifically, disclosed is a method for preventing onset of type 1 diabetes in an individual by administering to said individual at least one species of bacteria of the clostridial phylogenetic cluster IV (*Clostridium leptum* group).

## DESCRIPTION OF THE DRAWINGS

**Figure 1.** Normoglycemic female NOD mice who later progress to diabetes have elevated glucose stimulated plasma insulin and diminished lipids at an early age.

**a,** Incidence of diabetes in female and male NOD mice included in the longitudinal lipidomics study. The cumulative incidence of diabetes in this study was lower than the colony incidence of 80% in females and 35% in males. **b**, Age-dependent progression of lipidomic profiles in females, viewed as ratios of mean lipid concentrations of diabetes progressors (*n*=12) *vs.* non-progressors (*n*=14). The hierarchical clustering was performed across all 733 samples analyzed. PC, phosphatidylcholine; lysoPC, lysophosphatidylcholine. **c,** Blood glucose levels in 10-week-old female NOD progressors (*n*=11) and non-progressors (*n*=14) after 4 h fast and 5 minutes after intraperitoneal (i.p.) glucose (1 g/kg) administration (2-way ANOVA with glucose administration and diabetes progression as factors, reported P-value for diabetes progression; error bars ± SEM). **d,** Plasma insulin concentrations (mice and statistic same as in panel c**)**. **e**, There were no differences in body weight between the groups. **f**, Concentration of serum lysophosphatidylcholine (lysoPC; measured as total added concentration of PC(16:0/0:0) and PC(18:0/0:0)) in 8-week female NOD mice as dependent on diabetes progression and insulin autoantibody (IAA) positivity. Surrogate marker derived from lysoPC level and IAA positivity (Supplementary Fig. 3) was used to stratify mice according to diabetes risk in subsequent studies where mice were sacrificed for tissue-specific studies.

**Figure 2.** Lipid changes observed in children who later progress to T1D are also characteristic of the early prediabetic changes in female NOD mouse progressors.

**a,** Structure of the Hidden Markov Model (HMM) used in this study. The model is made to focus on progressive changes of lipidomic profiles over time[15] by assuming that returning back in states is not possible after state 2. Separate HMM models were developed for NOD female progressors and non-progressors. The nodes in the graph represent the hidden states, each of which emits a multivariate profile of metabolite concentrations, and arrows represent possible transitions between the states. **b**, HMM state progression as a function of age is similar for progressors and non-progressors. Each column shows the probabilities of being in the three states at a certain age, estimated by bootstrap. **c**, Differences in lipidomic profiles (mean lipid concentrations) between progressors and non-progressors as a function of the progressive metabolic state, colored according to bootstrap-based confidence intervals. **d**, Differences in lipid concentrations in diabetes progressors *vs.* nonprogressors that generalize across the species. Mapping shown on the left is inferred from longitudinal lipidomic profiles from DIPP study children[2] (Supplementary Fig. 2) and NOD female mice.

**Figure 3.** Female NOD mice at high risk of diabetes have more insulitis, elevated levels of insulinotropic amino acis in pancreatic islets, and diminished diversity of *C. leptum* bacteria in caecum.

**a,** Grading of pancreatic islet insulitis in normoglycemic 19-week-old female NOD mice comparing high- and low-risk groups. Insulitis was graded: 0, no visible infiltration, I peri-insulitis, II insulitis with < 50% and III insulitis with > 50% islet infiltration. 52 islets from 4 non-progressors (11-17 islets/each) and 28 islets from 3 non-progressors (7-10 islets/each) were graded. There was a tendency to more severe insulitis in the progressors *(P* = 0.07, $\chi^2$ test). **b**, Significantly regulated and selected other metabolites (*P*<0.07), out of 125 measured, in islets from female mice at high (HR) *vs.* low risk (LR) of developing diabetes. Fourteen mice were 8 weeks old (two IAA+ LR, three IAA- LR, four IAA+ HR, five IAA- HR) and 11 were 19 weeks old (four IAA+ LR, three IAA- LR, one IAA+ HR, three IAA- HR) at time of sacrifice. FDR (Max. *q*-value[47] at P<0.05) = 0.12. **c,** Bacterial diversity of caecum samples from 19-week old female NOD mice, four from HR group and seven from LR group, as detected with group specific DGGEs. Bifidobacteria did not amplify from any sample.

**Figure 4.** Markers of insulin resistance in 8-11 week old female NOD mice.

**a,** Glucose-stimulated insulin secretion is elevated in the high-risk (HR) group *(n* = 18) as compared to low-risk (LR) group *(n* = 12) (measured in NOD Study 3). **b**, No significant differences between the HR and LR group were found in glucose tolerance test (GTT) or **c,** insulin tolerance test (ITT) (measured in Study 3). **d**, The HR mice at 10 weeks of age have slightly more insulitis. Total 678 islets from 8 LR mice (60-123 islets/each) and 633 islets from 8 HR mice (59-102 islets/each) were graded as in Fig. 3a. **e**, Plasma leptin (combined Studies 3 and *4; n* = 24 for LR and *n* = 43 for HR) and **f**, adiponectin (Study 4; *n* = 14 for LR and *n* = 27 for HR) are elevated in 10-week-old HR mice.

**Supplementary Figure 1.** Lipidomic profiles of male NOD progressors do not differ from non-progressors.
Age-dependent progression of lipidomic profiles in NOD male mice, viewed as ratios of mean lipid concentrations of diabetes progressors (*n*=7) *vs.* the non-progressors *(n*=6*).* The hierarchial clustering was performed across all 439 samples analyzed.

**Supplementary Figure 2.** Metabolic states derived from hidden Markov Model in children who later progress to type 1 diabetes.
**a.** Distribution of HMM states as a function of age in DIPP children, shown separately for progressors and non-progressors. The HMM was derived from previously obtained data[1b] from diabetes progressors (*n*=56) and non-progressors (*n*=73), comprising a total of 1196 samples. **b** Progressopn of lipidomics states to diabetes in DIPP study children. Differences in mean lipid concentrations between progressors and non-progressors are shown for each of the three states.

**Supplementary Figure 3.** Surrogate marker for stratifying female NOD mice into two groups with high- and low-risk of developing autoimmune diabetes.
The marker is derived from lysophosphatidylcholine and IAA measurement from 8 week old female mice (same as shown in Figure 3.). The following algorithm was applied:

- Calculate lysoPC concentration (μmol/l) as a sum of concentrations of PC(16:0/0:0) and PC(18:0/0:0)
- Scale the lysoPC concentration to zero mean and unit variance → $lysoPC_S$
- Marker calculation

  o If IAA-, then Marker = $lysoPC_S$
  ◦ If IAA+, then Marker = $-lysoPC_S$

- Estimation of progressors (P) and non-progressors (NP)

  o If marker ≥ -0,1 then P, else NP

**Supplementary Figure 4.** Insulin resistance, weight, and adiposity in progression to T1D. **a.** Homeostatic model assessment (HOMA) index in high- vs. low-risk group (Study 4). **b.** Body weight and**c.** adipose tissue mass in the same groups.

**Supplementary Figure 5.** Microbial composition of caecum in 19-week-old female NOD mice, comparing- high and low-risk groups.
Principal Components Analysis plot of the composite DGGE dataset, which was calculated based DGGE profiles of predominant bacteria, *E. rectal - B. coccoides* group, C. *leptum* group, *Bacteroiden* spp. and *Lactobacillus-group,* bifidobacteria didn’r amplify. Star = high diabetes risk, dot= low diabetes risk.

**Supplemenray Figure 6.** Metabolic dysregulation in early type I diabetes pathogenesis - a summary of findings.

**DEFINITIONS**

In this connection Bacteria of the Clostridium leptum group - Clostridial phylogenetical cluster IV, which includes e.g. *Acetanaerobacterium elongatum*, *Anaerophilum agile, Anaerofilum pentosovorans, Anaerotruncus colihominis, Butyricoccus pullicaecorum*, *Clostridium cellulosi, Clostridium leptum, Clostridium methylpentosum*, *Clostridium orbiscindens, Clostridium sporosphaeroides, Clostridium viride, Ethanoligenes harbinense, Eubacterium desmolans, Eubacterium siraeum, Eubacterium plautii, Faecalibacterium prausnitzii, Hydrogenoanaerobacterium saccharovorans, Oscillibacter valericigenes, Papillibacter cinnamivorans*, *Pseudoflavonifractor capillosus, Ruminococcus albus, Ruminococcus bromii*, *Ruminococcus callidus, Ruminococcus flavefaciens, Sporobacter termitidis*, *Subdoligranulum variabile* and other closely related yet uncultured bacteria
In this connection "non-pathogenic bacteria" means bacteria that is not harmful to an individual. Especially bacteria naturally occurring in the intestinal flora of a healthy individual are preferred.
In this connection phrase "diminished diversity of *Clostridium leptum* group bacteria" means statistically significant difference between healthy individuals and those with disease (e.g. 17 amplicons against 12 amplicons, respectively).
In this connection "metabolite" is preferably a lysophospholipid (e.g., lysophosphatidylcholines such as lysoPC(16:0), lysoPC(18:0)), sphingosine-1-phosphate, arachidonic acid, arachidonic acid derived lipid mediators (e.g.

prostaglandin E2 or leukotrienes LTA4, LTB4), docosahexanoic acid eicosapentaenoic acid derived lipid mediators (e.g. resolvins such as Resolvin E1 and Resolvin E2, maresins, protectins), branched chain amino acids (valine, isoleucine, leucine) and their ketoacids (e.g. ketoleucine), glutamic acid, .

In this connection phrases "elevated / decreased metabolite level compared to healthy individual" means that concentration of a metabolite is above / below a set threshold defined in relation to the mean concentration in healthy individual with no islet autoantibodies who are not considered at-risk of type 1 diabetes.

In one embodiment of the invention the diversity of *Clostridium leptum* group bacteria in a sample is determined as follows:

(a) DNA is extracted from a fecal sample

(b) PCR is performed with primers 5'-GCACAAGCAGTGGAGT-3' and 5'-CGCCCGGGGCGCGCCCCGGGCGGGGCGGGGGCACGGGG GGGTTTTRTCAACGGCAGTC-3' in a total volume of 30 μl containing 1 μl of appropriately diluted template DNA, 0.4 μM of both primers, 0.2 mM dNTP, 1.25 units of Taq polymerase (Invitrogen) in a reaction buffer with 20 mM Tris-HCl (pH 8.4), 50 mM KCl, and 2 mM MgCl2. The PCR program consists of: initial denaturing at 94oC for 5 min, followed by 30 cycles of denaturing at 94oC for 45 s, primer annealing at 60oC for 30 s and elongation at 72oC for 60 s, and a final extension for 30 min at 72oC;

(c) DGGE analysis is performed using the Dcode Universal Mutation Detection System (BioRad) maintained at 60oC and 85 V for 16 h in 0.5 x TAE buffer (20 mM Tris-acetate, 0.5 mM EDTA, pH 8.0). Samples are loaded onto 8% acrylamide-bisacrylamide (37.5:1) gels with linear denaturing gradients from 30 to 60% (where 100% is 7 M urea and 40% (vol/vol) deionized formamide). The gels are stained with SYBR Green I (Molecular Probes) for 20 min at room temperature and the images are captured with a Gel Doc XR Gel Documentation System (BioRad);

(d) The DGGE gels are analyzed with the BioNumerics software (Applied Maths BVBA);

wherein diminished diversity of *Clostridium leptum* group bacteria indicates an increased risk of type 1 diabetes.

The determination of the serum metabolite can be followed up at several ages of the individual, preferably a child, and the result is compared to control groups of the same age as the child to be diagnosed. Also several serum metabolites can be determined for the child to be diagnosed, and the levels are compared to the levels of said metabolites for control groups.

In one embodiment the forementioned monitoring of one or more serum metabolites is combined with monitoring of emergence of autoimmunity in the child and / or determination of genetic risk for development of type 1 diabetes.

**Longitudinal serum lipidomics in pre-diabetic NOD mice**

Our first objective was to validate whether the NOD mouse was a good model of type 1 diabetes able to recapitulate the lipidomic-based metabolic phenotypes observed in the longitudinal study of children who later progressed to T1D (Type 1 Diabetes Prediction and Prevention project; DIPP)[2,8]. Hence we performed a murine study using NOD mice and recapitulated the protocol used in human studies (Study 1). A total of 70 NOD/Bom mice (26 female) were monitored weekly with serum collection from age 3 weeks until either (a) the development of diabetes (progressor group), or (b) followed until 36 weeks of age in females and 40 weeks in males in the absence of a diabetic phenotype (non-progressor group) (Fig. 1a). Similarly as in the DIPP study[2], we were primarily interested in early pre-diabetic differences of lipidomic profiles, in mice of the same colony, between diabetes progressors and non-progressors.

Lipidomic analysis using Ultra Performance Liquid Chromatography (UPLC) coupled to mass spectrometry (MS)[2] was performed on a complete sample series from 26 female mice (12 progressors, 14 non-progressors) and 13 male (seven progressors, six non-progressors) mice, comprising a total of 1172 samples or 30 samples/mouse on an average (733 samples from female and 439 from male mice), with 154 lipids measured in each sample. When comparing the lipid concentrations of diabetes progressors and non-progressors, the first weeks of life (3-10 weeks) were characterized by an overall lipid-lowering trend among the female progressors, while the period close to the disease onset (15 week and older) was characterized by elevated triacylglycerols and phospholipids (Fig. 1b). No such changes were observed in male mice (Supplementary Fig. 1). The NOD female progressors had similar levels of glycemia (Fig. 1c) than the non-progressors but to our surprise the progressors exhibited higher fasting as well as glucose-stimulated plasma insulin levels (Fig. 1d) despite the fact that no body weight differences were evident between progressors and non-progressors at 10 weeks of age (Fig. 1e). Together, these results imply that the mice who later progress to diabetes are characterized by enhanced glucose-stimulated insulin secretion (GSIS) at an early age or that they are inappropriately insulin resistant for their degree of body weight. In fact this increased GSIS associated to early evolutive stages towards type 1 diabetes is consistent with our earlier findings indicating that the children who later progress to diabetes are characterized by low serum ketoleucine and elevated levels of the more insulinotropic aminoacid leucine prior to seroconversion to insulin autoantibody (IAA) positivity[2,14].

**Mapping of human and NOD mouse pre-diabetic lipidomic profiles**

In order to systematically investigate similarities between of early metabolic phenotypes of autoimmune diabetes progressors in mice and men, we proceeded with comparative analysis of longitudinal lipidomic profiles from female NOD mice and DIPP study children[2]. However, given the sensitivity of metabolome to both genetic and environmental factors[6] and variable disease penetration the lipidomic profiles may individually change at different paces. We recently introduced a concept that the maturation of metabolic profiles with age, such as during normal development or early disease pathogenesis, can be described in terms of *metabolic states* derived using the Hidden Markov Model (HMM) methodology[15]. Instead of observing progression of average lipidomic profiles (Fig. 1b), our approach allows for each individual's lipidomic profiles to mature at its own pace. Such individual profiles are captured into a set of progressive HMM states (described by mean lipid profiles) using an underlying statistical model.

Here we applied the HMM methodology to study the longitudinal lipidomic profiles of female NOD mice (Fig. 2a) and identified a three-state HMM to describe the progression of metabolic states at early ages (3-10 weeks) (Fig. 2b). The first two states, corresponding to mean ages of approximately 4 weeks and 6 weeks, respectively, were characterized by decreased phospholipids and triacylglycerols among the progressors (Fig. 2c). A three-state HMM model of similar characteristics to the murine model was also applied to the complete lipidomics data from the longitudinal study of children who progressed to T1D[2]. The nested case-control study included 56 T1D progressors and 73 matched non-progressors, comprising a total of 1196 samples or 9.3 samples per child on average between birth and diabetes onset (in progressors). As in female NOD mice, the first state corresponding to first year of life was characterized by low triacylglycerols and specific phospholipids (Supplementary Fig. 2).

The similarity of state progression in children (Supplementary Fig. 2) and female NOD mice (Fig. 2c) presenting with diabetes suggests that the early disease stages as reflected in the lipidomes share similar metabolic perturbations. However, it is always a challenge to compare species which exhibit differences in systemic lipid metabolism as well as diet related effects on the lipidomic profiles. Consequently the mapping of molecular lipids between mouse and man may not be trivial. In order to compare progression of mouse and human lipidomic profiles we applied a new mapping algorithm that captures their dependencies across the two species[16]. By using this strategy it is possible to compare lipidomic profiles across the species, and we sought the disease effect by a two-way analysis on progressors/non-progressors *vs.* men/mice. We uncovered associations of functionally and structurally related lipids between the species (Fig. 2d) and confirmed strong association of diminished phospholipids with the development of the disease at an early age (HMM state 1). We can thus conclude that the lipid changes seen in children prior to the first seroconversion to islet autoantibodies are also characteristic of the early changes in female NOD mice progressors.

**Lysophosphatidylcholine and IAA in early diabetes progression**

Seroconversion to islet autoantibody positivity is associated with transiently elevated lysophosphatidylcholine (lysoPC) serum levels in children who subsequently progress to TID[2]. Here we measured the IAA levels in NOD mice at 8 weeks of age and similarly confirmed that the IAA-negative (IAA-) progressor female NOD mice had elevated lysoPC as compared to IAA- non-progressors (Fig. If). Intriguingly, IAA positivity had the opposite association with diabetes progression since the IAA-positive (IAA+) mice with high lysoPC were protected from diabetes (Fig. 1f). It can be speculated that due to their opposite association with disease progression IAA measurement in combination with lysoPC may help stratify the NOD mice according to their risk of developing diabetes. We derived a surrogate marker by combining autoantibody positivity and lysoPC concentration, which reasonably well discriminated between progressors and non-progressors ($\chi^2$=5.75, $P_{\chi^2}$=0.0044; Supplementary Fig. 3), with the NOD mice in the assigned "High-risk" group being at 4.3-fold higher risk (95% lower tolerance bound = 2.6, as calculated from 1000-fold resampling) of developing autoimmune diabetes as compared to the mice in the "Low-risk" group.

**Specific islet and liver pathways associate with T1D risk**

In an independent experiment normoglycemic female NOD mice from the same colony as in the first experiment were sacrificed at 8 (n=57) or 19 (*n*=14) weeks of age and blood, liver and pancreas samples were collected (Study 2). We selected sixteen 8-week-old mice (seven were IAA+) and thirteen 19-week-old mice (six were IAA+) for UPLC/MS based serum lipidomics analysis for subsequent risk stratification using the algorithm described above. Mice at high risk of developing diabetes showed a tendency towards more severe insulitis (Fig. 3a). In parallel liver and islet transcriptomics was performed for 19-week-old mice. When comparing high- and low-risk mice, independent of IAA level, the pathway analysis of islet gene expression data using Gene Set Enrichment Analysis (GSEA)[17] expectedly revealed upregulation of several apoptotic and immunoregulatory pathways in the high-risk group (Table 1). These pathways were associated with the autoimmune status, as they were also upregulated when comparing IAA+ and IAA- mice independent of diabetes risk. Some of the upregulated gene products of these pathways are known to be implicated in progression to autoimmune diabetes, including CD3 from the CTLA4 pathway[18], pro-inflammatory chemokine CCL5 (or RANTES) from the toll like receptor signalling pathway[19,20] and the IL-7 pathway[21].

**Table 1. Pathway analysis in female NOD mouse islets.**

Up to 10 most significantly affected pathways are shown at False Discovery Rate (FDR) $q<0.25$ for two different comparisons: (1) High (HR) *vs.* low diabetes risk (LR) and (2) IAA positive LR *vs.* other. Transcriptomics was performed in the islets of $n=10$ 19-week old female NOD mice (three IAA+ LR, two IAA- LR, two IAA+ HR, three IAA- HR). N, number of genes in the pathway; NES, normalized enrichment score; Source, gene list source.

| | N | NES | FDR *q* | Source |
|---|---|---|---|---|
| **Upregulated in progressors, associated with IAA positivity** | | | | |
| HIVNEFPATHWAY | 53 | 2,32 | 0,000000 | BioCarta |
| HSA04650_NATURAL_KILLER_CELL_MEDIATED_CYTOTOXICITY | 94 | 2,21 | 0,000157 | KEGG |
| APOPTOSIS_GENMAPP | 40 | 2,18 | 0,000307 | GenMAPP |
| CELL_CYCLE_KEGG | 79 | 2,16 | 0,00041 | GenMAPP |
| HSA04660_T_CELL_RECEPTOR_SIGNALING_PATHWAY | 90 | 2,15 | 0,000413 | KEGG |
| IL7PATHWAY | 16 | 2,04 | 0,00166 | BioCarta |
| APOPTOSIS | 63 | 2,03 | 0,001676 | GenMAPP |
| CTLA4PATHWAY | 16 | 2,02 | 0,0019 | BioCarta |
| HSA03022_BASAL_TRANSCRIPTION_FACTORS | 30 | 2 | 0,002515 | KEGG |
| HSA04662_B_CELL_RECEPTOR_SIGNALING_PATHWAY | 59 | 2 | 0,002531 | KEGG |
| **Upregulated in progressors, not associated with IAA positivity** | | | | |
| **HSA00240_PYRIMIDINE_METABOLISM** | 82 | 2,12 | 0,000763 | KEGG |
| RIBOSOMAL_PROTEINS | 71 | 2,03 | 0,001743 | GenMAPP |
| HSA04610_COMPLEMENT_AND_COAGULATION_CASCADES | 62 | 1,92 | 0,004885 | KEGG |
| NKCELLSPATHWAY | 18 | 1,9 | 0,005527 | BioCarta |
| CARM_ERPATHWAY | 24 | 1,89 | 0,006187 | BioCarta |
| HSA00100_BIOSYNTHESIS_OF_STEROIDS | 21 | 1,88 | 0,006819 | KEGG |
| HSA00230_PURINE_METABOLISM | 135 | 1,87 | 0,00801 | KEGG |
| KREBS_TCA_CYCLE | 28 | 1,85 | 0,009439 | GenMAPP |
| INTRINSICPATHWAY | 22 | 1,82 | 0,011212 | BioCarta |
| GLYCOLYSIS_AND_GLUCONEOGENESIS | 38 | 1,81 | 0,012117 | GenMAPP |
| **Downregulated in IAA positive non-progressors** | | | | |
| OXIDATIVE_PHOSPHORYLATION | 56 | -1,76 | 0,080640 | GenMAPP |
| KREBS_TCA_CYCLE | 28 | -1,66 | 0,129344 | GenMAPP |
| MITOCHONDRIAL_FATTY_ACID_BETAOXIDATION | 15 | -1,61 | 0,152160 | GenMAPP |
| HSA03010_RIBOSOME | 55 | -1,57 | 0,163501 | KEGG |
| HSA00480_GLUTATHIONE_METABOLISM | 34 | -1,45 | 0,196112 | KEGG |
| HSA00280_VALINE_LEUCINE_AND_ISOLEUCINE_DEGRADATION | 40 | -1,46 | 0,196296 | KEGG |
| **Upregulated in IAA positive non-progressors** | | | | |
| ST_INTEGRIN_SIGNALING_PATHWAY | 78 | 2,06 | 0,000774 | STKE |

(continued)

| | | | | |
|---|---|---|---|---|
| **Upregulated in IAA positive non-progressors** | | | | |
| HSA05211_RENAL_CELL_CARCINOMA | 67 | 1,99 | 0,001681 | KEGG |
| INTEGRIN_MEDIATED_CELL_ADHESION_KEGG | 90 | 1,95 | 0,002570 | GenMAPP |
| IL6PATHWAY | 19 | 1,95 | 0,002717 | BioCarta |
| SA_PTEN_PATHWAY | 16 | 1,85 | 0,005938 | SigmaAldrich |
| ST_INTERLEUKIN_4_PATHWAY | 23 | 1,84 | 0,006384 | STKE |
| SIG_CHEMOTAXIS | 44 | 1,77 | 0,011176 | SignalingAlliance |
| CELL_GROWTH_AND_OR_MAINTENANCE | 58 | 1,75 | 0,012905 | GO |
| ECMPATHWAY | 20 | 1,73 | 0,015894 | BioCarta |
| RAC1PATHWAY | 22 | 1,68 | 0,021239 | BioCarta |

Several upregulated pathways in high-risk mice were not associated with the IAA titer. These pathways associated with high risk of developing diabetes were mainly metabolic pathways and included upregulated genes from TCA cycle and glycolysis/gluconeogenesis (Table 1). In order to directly measure the metabolic products of these pathways, we performed metabolomic analysis of islets using two-dimensional gas chromatography coupled to time-of-flight mass spectrometry (GCxGC-TOFMS)[22]. Metabolomics confirmed dysregulation of energy and amino acid metabolism in the islets of high-risk mice (Fig. 3b), as several key metabolites of these pathways were found upregulated, including glutamic and aspartic acids, as well as at a marginal significance level all three branched chain amino acids (BCAAs). These elevated amino acids are known insulin secretagogues in β-cells[23]. The top ranking gene in this pathway, Glucose-6-phosphatase, catalytic, 2 (G6PC2; fold change high- *vs*. low-risk group +11%, $P$=0.0034*),* controls the release of glucose from liver into the bloodstream. However, the animals included in this study, as in the earlier longitudinal study, were normoglycemic and there were no differences in body weight between the two groups. The metabolic changes in β -cells and liver can thus explain the observed elevated GSIS in mice at high risk for developing autoimmune diabetes (Figs. 1c-e).

**Markers of insulin resistance in progression to T1D**

There is evidence from clinical studies that insulin resistance is a risk factor for progression to T1D[24,25]. It is also known that the NOD genetic background may predispose the mice to insulin resistance[26]. To test for insulin resistance as a potential explanation for the observed metabolic phenotype of high-risk mice, we performed two independent studies in another NOD colony where (Study 3) $n$ = 36 female NOD/MrkTac mice were tested for GSIS, glucose and insulin tolerance, and plasma leptin between 8 and 11 weeks of age; and (Study *4)* $n$ = 42 female NOD/MrkTac were sacrificed at 10 weeks of age and tested for insulitis, plasma leptin and adiponectin. As before, serum lipidomics and IAA assays were performed to stratify the mice into high- and low-diabetes-risk groups.

We confirmed the elevated GSIS in high risk mice (Fig. 4a) but found no significant difference in glucose responses to intraperitoneal glucose or insulin between the groups (Figs. 4b-c), in the Homeostatic model assessment (HOMA-IR) index or GLUT4 expression in white adipose tissue and muscle (Supplementary Fig. 4a-c). In agreement with the results from older mice (Fig. 3a), the 10-week old female NOD mice at higher risk of developing diabetes have already signs of more insulitis than their low-risk counterparts, although the average degree of insulitis is mild in both groups (Fig. 4d). Surprisingly, the adipose tissue derived hormones leptin (Fig. 4e) and adiponectin (Fig. 4f) were both elevated in plasma of high-risk mice despite no significant differences in weight or adiposity (Supplementary Fig. 4d-f).

**Diminished diversity of gut microbiota associates with diabetes risk**

We recently found that serum metabolome of germ-free mice is similar to pre-autoimmune metabolomes of children who later progress to T1D[27], thus implying that gut microbiota of T1D progressors may be devoid of important constituents or has an impaired function that predisposes the children to T1D. Given the observed similarities of metabolomes of diabetes progressors in mice and men (Fig. 2), we found that the observed metabolic differences between the high- and low-risk mice may be reflected in differences of their gut microbial composition.

We characterized the microbial composition of caecum samples from high- and low-risk mice from Study 2 using denaturing gradient gel electrophoresis (DGGE) as previously described[28,29]. We indeed found that the total bacterial composition was more coherent in low-risk mice than in the high-risk mice (Supplementary Fig. 5) and bacteria of high-risk mice had significantly diminished diversity of the *Clostridium leptum* group of the *Firmicutes phylum* (Fig. 3c).

**IAA positivity and protection from autoimmune diabetes**

Given that the metabolic profile is normalized in children following the seroconversion[2], we proposed earlier that generation of autoantibodies may be a physiological response to early metabolic disturbances. In the present study (mice from Study 2), we investigated the pathways in the IAA+ low-risk female mice and compared them to all other groups. The IAA+ low-risk mice were characterized by several elevated signaling pathways in the islets including the IL-4 and IL-6 pathways (Table 1). IL-4 is known to be protective from diabetes in NOD mouse[30]. Conversely IAA+ low-risk mice had reduced expression of pathways mainly related to mitochondrial function and TCA cycle, BCAA catabolism, beta oxidation and oxidative phosphorylation. It is unclear how downregulation of these pathways may protect against T1D. However downregulation of these pathways will lead to a state of reduced production of reactive oxygen species (ROS)[31] which may explain at least in part the conserved β -cell functionality. This would offer a potential protective mechanism linking decreased ROS production to the prevention of β-cell apoptosis in IAA+ mice which do not progress to diabetes. Our results stress the need for similar studies in terms of protection from diabetes in individuals who seroconverted but did not progress to overt disease.

This study emphasize the translatability of the our previous findings from the large-scale clinical study[2] into the tissue-specific context. Also, our study highlights that specific metabolic disturbances are identifiable early on during the evolutive stages and could potentially be linked to pathogenic mechanisms implicated in the progression to autoimmune diabetes (Supplementary Fig. 6).

Our study implicates that diminished diversity of specific bacterial groups such as C. *leptum* is associated with the early metabolic disturbances. The fact that the diabetes-associated differences in microbial composition were observed among the mice of the same colony suggests that the observed diminished microbial diversity is likely a consequence of immunological or metabolic response. Microbial communities are sensitive to disturbances and may subsequently not return to the their original state[44] and diminished diversity of gut microbiota has been found e.g. in obesity[45]. Interestingly, diminished diversity of the anti-inflammatory commensal bacterium *Faecalibacterium prausnitzii* from the C. *leptum* subgroup characterizes also Crohn's disease[46].

The invention is illustrated by the following non-limiting examples. It should be understood, however, that the embodiments given in the description above and in the examples are for illustrative purposes only, and that various changes and modifications are possible.

## EXAMPLES

### Experimental animals and sample collection

All experimental procedures were approved by the Committee for Laboratory Animal Welfare, University of Turku. The mice were kept in an animal room maintained at 21 $\pm$ 1°C with a fixed 12:12 h light-dark cycle. Standard rodent chow (Special Diet Services, Witham, UK) and water were available ad libitum. The colonies of NOD/Bom mice used were bred and maintained in the animal facilities of University of Turku and originated from mice purchased from Taconic Europe (Ry, Denmark). 26 female and 44 male NOD mice (Study 1) underwent weekly blood sampling by venopuncture from the tail vein starting at 3 weeks of age until the mice developed diabetes (blood glucose $\geq$ 14.0 mmol/ in two consecutive weeks) or until female mice reached 36 weeks and male mice 40 weeks of age. Serum was separated and quickly frozen in -70°C for metabolomic analysis. Blood samples for detection of insulin autoantibodies (IAA) were collected from tail vein at the age of 8 weeks. Plasma samples for insulin were collected between noon and 2 PM after 4 h fast and two days later 5 minutes after intraperitoneal glucose (1 g/kg) administration at the age of 10 weeks. Another set of euglycemic NOD/Bom female mice (Study 2) was sacrificed with decapitation under CO2 anesthesia at the age of 8 weeks (n = 57) or 19 weeks (n = 14), and blood, liver and pancreas samples were collected.

Two separate batches (n = 36 and 42, Studies 3 and 4) of female NOD/MrcTac were delivered from Taconic USA (Hudson, NY, USA) at 5 weeks of age. In Study 3, intraperitoneal glucose tolerance test was performed after 4 h fast at 8 weeks of age by administering glucose (10% [wt/vol], 1 g/kg body weight) and measuring tail vein blood glucose and serum insulin. Serum samples for lipidomics and IAA were collected from tail vein at 10 weeks of age. Intraperitoneal insulin tolerance test was performed after 1 h fast at 11 weeks of age by administering human insulin (1.0 IU/kg body weight, Protaphane, Novo Nordisk, Bagsvaerd, Denmark). In Study 4, mice were sacrificed at 10 weeks of age after 4 h fast by cardiac puncture under anesthesia. Gonadal white adipose tissue (WAT) depot was carefully dissected and weighted, and was used as a marker of adiposity. Serum samples for IAA, lipidomics and adipokine panel assays, gonadal WAT, gastrocnemius muscle and pancreas samples were collected, and stored at -70°C until analyses. HOMA-IR, an estimate of insulin resistance, was calculated as fasting insulin ($\mu$IU/ml) $\times$ fasting glucose (mmol/l)/22.5. Statistical significances were analyzed with Student's t-test or two-way ANOVA using GraphPad Prism 4.

### Plasma glucose, insulin, leptin and adiponectin

Blood glucose was measured with Precision Xtra™ Glucose Monitoring Device (Abbott Diabetes Care, IL). Plasma insulin was analyzed with Mouse Ultrasensitive ELISA kit (Mercodia, Uppsala, Sweden) or together with leptin with Milliplex Mouse Adipokine Panel (Millipore, Billerica, MA, USA). Plasma adiponectin was measured with Mouse Adiponectin ELISA kit from Millipore.

### Islet isolation

Pancreatic islets were isolated using Ficoll 400 (Sigma-Aldrich, St Louis, MI, USA) gradient method[1a]. In brief, the pancreata were incubated with Collagenase P (0,5 mg/ml, Roche Diagnostics, Mannheim, Germany) in HBSS containing 10 mM HEPES, 1 mM $MgCl_2$, 5mM Glucose, pH 7.4 for 17 min. After two rounds of washing, the pellet was resuspended in Ficoll 25%, and the densities 23%, 20% and 11% were layered on top. After centrifuge, the islet layer between densities 23% and 20% was collected and washed twice before snap_freezing the pellet for metabolomics or homogenization in lysis buffer for RNA extraction. Samples were stored in -70°C until analyses.

## Histopathology of diabetes

Pancreata from euglycemic NOD mice were cryosectioned. 5 μm sections with > 20 μm intervals were stained with hematoxylin & eosin and graded for insulitis as follows: 0, no visible infiltration, I peri-insulitis, II insulitis with < 50% and III insulitis with > 50% islet infiltration. Total 678 islets from 8 female 10-week-old low-risk mice (60-123 islets/each) and 633 islets from 8 high-risk mice (59-102 islets/each), and 52 islets from 4 female 19-week-old low-risk mice (11-17 islets/each) and 28 islets from 3 high-risk mice (7-10 islets/each) were graded. Statistical significance was analyzed with Student's t-test or Chi Square test using GraphPad Prism 4.

## IAA assay

Murine IAA were measured by a radiobinding microassay (RIA) with minor modifications to that previously described for human IAA[2a]. Mouse sera (2.5 μl) and serial dilutions of standard samples (5 μl) of a serum pool obtained from persons with a high IAA titer were incubated for 72 h with 15,000 cpm mono$^{125}$I-(TyrA14)-insulin (Amersham, GE Healthcare, Buckinghamshire, UK) in the presence or absence of an excess of unlabeled human recombinant insulin (Roche Diagnostics, Mannheim, Germany). Antibody complexes were precipitated by adding 50 μl TBT buffer (50 mM Tris, pH 8,0, 0,1% Tween 20) containing 8 μl Protein A and 4 μl Protein G Sepharose (Amersham). After repeated washings the bound radioactivity was measured with a liquid scintillation detector (1450 Microbeta Trilux, Perkin Elmer Life Sciences Wallac, Turku, Finland). The specific binding was calculated by subtracting the non-specific binding (excess unlabeled insulin) from total binding and expressed in relative units (RU) based on standard curves run on each plate. The cut-off value for mouse IAA positivity was set at the mean + 3SDS in 16 BALB-mice, i.e. 1.79 relative units (RU).

## Lipidomic analysis

Serum samples (10 μl) in Eppendorf tubes were spiked with a standard mixture containing 10 lipid compounds at a concentration level of 0.2 μg/sample, and mixed with 10 μl of 0.9% sodium chloride and 100 μl of chloroform:methanol (2:1). After 2 min vortexing and 1 h standing the samples were centrifuged at 10000 rpm for 3 min and 60 μl of the lower organic phase was taken to a vial insert and spiked with 20 μl of three labelled lipid standards at a concentration level of 0.2 μg/sample.

The lipidomics runs were performed on a Waters Q-Tof Premier mass spectrometer combined with an Acquity Ultra Performance LC™ (UPLC; Milford MA). The solvent system consisted of 1) water with 1% 1M $NH_4Ac$ and 0.1% HCOOH and 2) LC/MS grade acetonitrile/isopropanol (5:2) with 1% 1M $NH_4Ac$, 0.1% HCOOH. The gradient run from 65% A / 35% B to 100% B took 6 min and the total run time including a 5 min reequilibration step was 18 min. The column (at 50 °C) was an Acquity UPLC™BEH C18 (1 × 50 mm, 1.7 μm particles) and the flow rate was 0.200 ml/min. The lipids were profiled using ESI+ mode and the data collected at a mass range of m/z 300-1200. The data was processed by using MZmine software (version 0.60)[3a,4a] and the lipid identification was based on an internal spectral library[5].

## Metabolomic analysis

Depending **on** the protein concentrations of PBS buffered cell solutions, 20-40 μL samples were taken for islet metabolomic analysis. 10 μL of an internal standard labeled palmitic acid-16,16,16-$d_3$ (250 mg/L) and 400 μL of methanol solvent were added to the sample. After vortexing for 2 min and incubating for 30 min at room temperature, the supernatant was separated by centrifugation at 10,000 rpm for 5 min. The sample was dried under constant flow of nitrogen gas and derivatized with 25 μL of MOX (1 h, 45 °C) and MSTFA (1 h, 45 °C). 5 μL of retention index standard mixture with five alkanes (125 ppm) was added to the metabolite mixture.

Islet samples were analyzed by two-dimensional gas chromatography coupled to time of flight mass spectrometry (GCxGC-TOF/MS). The instrument used was a Leco Pegasus 4D (Leco Inc., St. Joseph, MI), equipped with an Agilent GC 6890N from Agilent Technologies (Santa Clara, CA) and a CombiPAL autosampler from CTC Analytics AG (Zwingen, Switzerland). The modulator, secondary oven and time-of-flight mass spectrometer were from Leco Inc. The GC was operated in split mode with a 1:20 ratio. Helium with a constant pressure of 39.6 psig was used as carrier gas. The first dimension GC column was a non-polar RTX-5 column, 10 m x 0.18 mm x 0.20 μm (Restek Corp., Bellefonte, PA), coupled to a polar BPX-50 column, 1.50 m x 0.10 mm x 0.10 μm (SGE Analytical Science, Ringwood, Australia). The temperature program was as follows: initial temperature 50 °C, 1 min → 295 °C, 7 °C/min, 3 min. The secondary oven was set to 20 °C above the oven temperature. Inlet and transfer line temperatures were set to 260 °C. The second dimension separation time was set to 5 s. The mass range used was 45-700 amu and the data collection speed was 100 spectra/second. Raw data were processed using Leco ChromaTOF software, followed by alignment using in-house developed software Mylly. The metabolites were identified by using an in-house reference compound library together with The Palisade Complete Mass Spectral Library, 600K Edition (Palisade Mass Spectrometry, Ithaca, NY).

**Gene expression and pathway analysis**

**RNA extraction** from islets was carried out with Rneasy minikit (QIAGEN GmbH, Hilden, Germany) and from liver, skeletal muscle (m. gastrocnemius) and gonadal white adipose tissue with Trizol reagent (Invitrogen, Carlsbad, CA) followed by RNase-free DNase I treatment (QIAGEN GmbH) and purification with Rneasy minikit. Pancreatic islets and liver for microarray analysis were collected from 19-week-old euglycemic female NOD/Bom mice. Skeletal muscle and adipose tissue for GLUT4 mRNA expression were collected from 10-week-old female NOD/MrkTac mice.

**GLUT4 mRNA expression** in skeletal muscle and gonadal white adipose tissue was measured by quantitative real-time PCR. CDNA synthesis was performed with High Capacity RNA-to-cDNA Kit according to manufacturer's protocol. Real-time PCR was performed with 7300 Real Time PCR system, pre-designed TaqMan® Gene Expression Assay for GLUT4 and TaqMan® Endogenous Control Assay for β-actin. The 20 μl PCR reactions contained 8 μl cDNA, 8 μl TaqMan® Gene Expression Master Mix, 1 μl GLUT4 TaqMan Gene Expression Assay, 1 μl b-actin TaqMan Endogenous control Assay and 2 μl depc water. Cycling parameters for real-time RT-PCR were as follows: 50°C for 2 min, 95°C for 10 min followed by 40 cycles of 95 °C for 15 seconds and 60 °C for one minute. GLUT4 mRNA levels were expressed relative to β-actin, which was used as a housekeeping gene. Relative gene expression was calculated using the comparative CT method and $RQ=2^{-\Delta\Delta CT}$ formula. All reagens from Applied Biosystems (Foster City, CA, USA).

**RNA amplification** was performed from 300 ng total RNA with Ambion's (Austin, TX) Illumina RNA TotalPrep Amplification kit (cat no AMIL1791). IVT reaction overnight (14 h), during it cRNA was biotinylated. Both before and after the amplifications the RNA/cRNA concentrations where checked with Nanodrop ND-1000 (Wilmington, DE) and RNA/cRNA quality was controlled by BioRad's Experion electrophoresis station (Hercules, CA).

**Hybridizations.** 1.50 μg each sample was hybridized to Illumina's MouseWG-6 Expression BeadChips, version 2 (BD-201-0602) at 58 °C overnight (18 h) according to Illumina Whole-Genome Gene Expression Direct Hybridization protocol, revision A. Hybridization was detected with 1 μg/ml Cyanine3-streptavidine, GE Healthcare Limited (Chalfont, UK) (cat no PA43001). Chips were scanned with Illumina BeadArray Reader, BeadScan software version 3.5. The numerical results were extracted with Illumina's GenomeStudio software v 1.0 without any normalization.

**Preprocessing.** Bead Summary data, exported from Illumina's GenomeStudio software, was preprocessed using *beadarray* package[6a] of R/Bioconductor[7a] as follows. Data was transformed to logarithm (base 2), and normalized using quantile method[8a], which equalizes the distribution of probe intensities across a set of microarrays.

**Pathway analysis.** Gene Set Enrichment Analysis (GSEA)[9a], a commonly used pathway analysis technique for microarray gene expression data analysis, uses a Kolmogorov-Smirnov like statistic to test whether selected gene sets are enriched among the most up or down regulated genes. Linear Models for Microarray Data (LIMMA) approach[10a] identifies differentially expressed genes by fitting a linear model to the expression data of each gene, and computing moderated t-statistic using posterior residual standard deviations to account for the gene-specific variability of expression values. Here, we used the R/Bioconductor packages[7a] and LIMMA[10a] for testing differential expression of genes. We then performed pre-ranked GSEA analysis using the moderated t-statistic for ranking the gene list, to test for enrichment of gene sets from a variety of pathway databases such as Gene Ontology (GO)[11a], GenMAPP[12a], BioCarta (http://www.biocarta.com), Signal Transduction Knowledge Environment (STKE) (http://stke.sciencemag.org/), and KEGG[13a] curated in Molecular Signatures Database (MSigDB)[9a].

**Clustering.** Leading edge genes of an enriched pathway are the genes that account for the enrichment signal[9a]. For selected pathways that are found statistically significant by GSEA, the pathway profiles are calculated as average expression of all leading edge genes. This matrix of pathway profiles of selected pathways was then augmented with selected metabolite profiles. Then the numerical values in this matrix were normalized with the autoantibody-negative non-progressors (IAA- & NP) *i.e.,* each numerical value of a variable is divided by the average values from IAA- & NP samples, and transformed to logarithmic (base 2) scale. Then the variables were scaled for unit variance. Finally, hierarchical clustering was applied using Euclidean metric and complete linkage method[14a] for computing inter-cluster distances. An R package called *gplots* (http://www.r-project.org/) was used for the clustering and displaying the numerical values as a heat map.

**Microbiological analysis**

DNA was extracted from 300 mg of fecal sample from caecum using FastDNA Spin Kit for Soil (QBIOgene, Carlsbad, CA,) with modifications to the manufacturer's instructions[15a]. PCR-DGGEs of predominant bacterial PCR-DGGE and group specific PCR-DGGEs (bifidobacteria, *Lactobacillus-group, Eubacterium rectale - Blautia coccoides* clostridial group (Erec-group), *Clostridium leptum* clostridial group (Clept group), and genus *Bacteroides)* were performed as described previously[16]. The comparison of the profiles and the quantification of the amplicons were performed using BioNumerics software version 5.1 (Applied Maths BVBA, Sint-Martens-Latem, Belgium). The statistical analysis of amplicon numbers was performed with the Student's t-test with unequal variances. Clustering was performed with Pearson correlation from each bacterial group besides using composite datasets in which amplicons with the total surface area of at least 1% were included in the similarity analysis. Principal component analysis was performed with the BioNumerics software.

**Basic statistical analyses**

R statistical software (http://www.r-project.org/) was used for data analyses and visualization. The concentrations were compared using the Wilcoxon rank-sum test, with p-values <0.05 considered statistically significant. To account for multiple comparisons, false discovery rates among significantly differing lipids were estimated using q-values[17a,18a]. False discovery rates were computed using the R package q-value. The fold difference was calculated by dividing the median concentration in progressors by the median concentration in nonprogressors and taking the base-2-log of the resulting value. This makes interpretation easy as values greater/smaller than zero correspond to up/down-regulated lipids in the progressor group. In clustering we applied a customized correlation based distance metric

$$d_{ij} = 1/\log(|cor(x_i, x_j)|),$$

where $x_i$ and $x_j$ denote the concentrations of lipids i and j in the sample set. Ward's method was then applied in hierarchical clustering using this distance measure[19a].

**Hidden Markov Model of metabolic state progression**

Metabolic state development in progressors and non-progressors was modeled by separate Hidden Markov Models[20a], making it possible to align individuals based on metabolic states instead of age, and to compare the metabolic states in progressors and no-progressors. The modeling assumptions under which the models are fitted to data are that individual mice share a similar developmental progression but the timing of the states may vary, and that metabolite profiles in each state may be different for progressors and non-progressors. Model fitting was done by the standard Baum-Welch algorithm using the MATLAB toolbox by Kevin Murphy (http://people.cs.ubc.ca/~murphyk/Bayes/hmm.html). The model structure was validated by the bootstrap in the same way as in our earlier studies[20a], and confidence intervals were estimated with non-parametric bootstrap (5000 samples).

**Mapping of human and mouse metabolites**

Let $X \in \mathbf{R}^{N \times D_X}$ and $Y \in \mathbf{R}^{M \times D_Y}$ be two data matrices with N and M samples, $M \geq N$, and dimensions $D_X$ and $D_Y$ respectively. The task is to find a permutation $p$ of samples in $Y$ such that each sample $x_i$ in $X$ is matched with $y_{p(i)}$ in $Y$, that is, we assume a one-to-one matching of samples between the two data matrices. Since the data matrices do not lie in the same data space, it is not possible to use distance as matching criterion. We have introduced a new methodology based on statistical dependencies between the data sets to solve this problem[21]. The idea is to compute from the data features or statistical descriptors that maximize statistical dependencies, and do the matching based on the descriptors. In practice, we will project the data onto a lower-dimensional subspace such that the statistical dependencies between the datasets are maximized, and find a matching of samples in this comparable subspace.

Let $f(x) = xw_x^T$ and $g(y) = yw_y^T$ be the descriptors, here chosen to be linear transformations ($x, w_x \in \mathbf{R}^{1 \times D_x}$ and $y, w_y \in \mathbf{R}^{1 \times D_y}$). Using correlation as a dependency measure we get the optimization problem $\max_{p, w_x, w_y} corr(Xw_x^T, Y(p)w_y^T)$; maximizing correlation amounts to maximizing mutual information for normally distributed data, and is generally a reasonable computational approximation.

We use an iterative algorithm to solve this optimization problem. In the first step the projections are computed given a fixed permutation $p$, and in the second step the permutations are optimized given fixed projections. These two steps are alternated until convergence. In practice, *canonical correlation analysis* can be used to compute projections given a permutation, and solving the permutation given fixed projections leads to an *assignment problem* which can be solved using the *Hungarian algorithm.*

The method described above can be used to find a matching given any pair of human-mouse datasets. The matching can be further improved by combining solutions obtained by several human-mouse dataset pairs to give a *consensus matching*. For simplicity we assume that the pairs of human-mouse datasets are independent samples, which holds approximately assuming a large number of datasets.

In order to combine different matching solutions, we create a contingency table based on all observed matching solutions. The lipids of the two species are the labels of rows and columns of the contingency table, and each cell gives the count of how many times the corresponding lipids in the two species have been paired in the observed matching solutions. Finding the consensus pairing given the contingency table reduces to finding a maximum bipartite matching between the rows and columns of the contingency table. This can again be solved by using the Hungarian algorithm.

**Bootstrap-based two-way analysis**

In order to find disease effects shared by NOD mice and humans in the DIPP study, we first paired metabolites of the two organisms, then estimated the metabolic states of progressor and non-progressor men and mice by HMMs, and finally did a bootstrap-based two-way analysis on progressors/non-progressors *vs.* men/mice to identify disease and organism effects and their interactions. The data-driven pairing or the metabolites and the four HMMs were computed as described above. The two-way analysis of disease effect was done by first removing the organism effect, represented with a single mean parameter estimated by least squares, and then computing bootstrap confidence intervals for the disease effect of pooled men and mice. Organism and cross effects were estimated analogously.

**REREFENCES**

1. Achenbach, P., Bonifacio, E., Koczwara, K. & Ziegler, A.-G. Natural history of type 1 diabetes. Diabetes 54, S25-31 (2005).
2. Oresic, M. et al. Dysregulation of lipid and amino acid metabolism precedes islet autoimmunity in children who later progress to type 1 diabetes. J. Exp. Med. 205, 2975-2984 (2008).
3. Gale, E. A. M. The rise of childhood type 1 diabetes in the 20th century. Diabetes 51, 3353-3361 (2002).
4. Harjutsalo, V., Sjöberg, L. & Tuomilehto, J. Time trends in the incidence of type 1 diabetes in Finnish children: a cohort study. Lancet 371, 1777-1782 (2008).
5. Patterson, C. C. et al. Incidence trends for childhood type 1 diabetes in Europe during 1989-2003 and predicted new cases 2005-20: a multicentre prospective registration study. Lancet 373, 2027-2033 (2009).
6. Oresic, M., Vidal-Puig, A. & Hänninen, V. Metabolomic approaches to phenotype characterization and applications to complex diseases. Expert Rev. Mol. Diagn. 6, 575-585 (2006).
7. Hagopian, W. A. et al. TEDDY - the environmental determinants of diabetes in the young: an observational clinical trial. Ann. NY Acad. Sci. 1079, 320-326 (2006).
8. Kupila, A. et al. Feasibility of genetic and immunological prediction of type 1 diabetes in a population-based birth cohort. Diabetologia 44, 290-297 (2001).
9. Anderson, M. S. & Bluestone, J. A. The NOD mouse: a model of immune dysregulation. Annu. Rev. Immunol. 23, 447-485 (2005).
10. Atkinson, M. A. & Leiter, E. H. The NOD mouse model of type 1 diabetes: as good as it gets? Nature 5, 601-604 (1999).
11. Chervonsky, A. V. Influence of microbial environment on autoimmunity. Nat. Immunol. 11, 28-35 (2010).
12. Singh, B. & Rabinovitch, A. Influence of microbial agents on the development and prevention of autoimmune diabetes. Autoimmunity 15, 209-213 (1993).
13. Wen, L. et al. Innate immunity and intestinal microbiota in the development of type 1 diabetes. Nature 455, 1109-1113 (2008).
14. Floyd, J. C. J., Fajans, S. S., Knopf, R. F. & Conn, J. W. Evidence that insulin release is the mechanism for experimentally induced leucine hypoglycemia in man. J. Clin. Invest. 42, 1714-1719 (1963).
15. Nikkilä, J. et al. Gender dependent progression of systemic metabolic states in early childhood. Mol. Syst. Biol. 4, e197 (2008).
16. Tripathi, A., Klami, A. & Kaski, S.,Using dependencies to pair samples for multi-view learning. In TKK Reports in Information and Computer Science, Report No. TKK-ICS-R8 (2008).
17. Subramanian, A. et al. Gene set enrichment analysis: A knowledge-based approach for interpreting genome-wide expression profiles. Proc. Nat. Acad. Sci. U S A 102, 15545-15550 (2005).
18. Keymeulen, B. et al. Insulin needs after CD3-Antibody therapy in new-onset type 1 diabetes. N. Engl. J. Med. 352, 2598-2608 (2005).
19. Eizirik, D. L., Moore, F., Flamez, D. & Ortis, F. Use of systems biology approach to understand pancreatic {beta} -cell death in type 1 diabetes. Biochem. Soc. Trans. 36, 321-327 (2008).
20. Zhernakova, A. et al. Genetic variants of RANTES are associated with serum RANTES level and protection for type 1 diabetes. Genes Immun. 7, 544-549 (2006).

21. Calzascia, T. et al. CD4 T cells, lymphopenia, and IL-7 in a multistep pathway to autoimmunity. Proc. Natl. Acad. Sci. USA 105, 2999-3004 (2008).
22. Welthagen, W. et al. Comprehensive two-dimensional gas chromatography time-of-flight mass spectrometry (GC-GC-TOF) for high resolution metabolomics: biomarker discovery on spleen tissue extracts of obese NZO compared to lean C57BL/6 mice. Metabolomics 1, 65-73 (2005).
23. Newsholme, P., Brennan, L. & Bender, K. Amino acid metabolism, {beta}-cell function, and diabetes. Diabetes 55, S39-47 (2006).
24. Fourlanos, S. et al. Insulin resistance is a risk factor for progression to Type 1 diabetes. Diabetologia 47, 1661-1667 (2004).
25. Xu, P. et al. Role of insulin resistance in predicting progression to type 1 diabetes. Diabetes Care 30, 2314-2320 (2007).
26. Chaparro, R. J. et al. Nonobese diabetic mice express aspects of both type 1 and type 2 diabetes. Proc. Natl. Acad. Sci. USA 103, 12475-12480 (2006).
27. Velagapudi, V. R. et al. The gut microbiota modulates host energy and lipid metabolism in mice. J. Lipid Res. 51, 1101-1112 (2010).
28. Maukonen, J. et al. Prevalence and temporal stability of selected clostridial groups in irritable bowel syndrome in relation to predominant faecal bacteria. J Med Microbiol 55, 625-633 (2006).
29. Maukonen, J., Matto, J., Suihko, M.-L. & Saarela, M. Intra-individual diversity and similarity of salivary and faecal microbiota. J. Med. Microbiol. 57, 1560-1568 (2008).
30. Rapoport, M. et al. Interleukin 4 reverses T cell proliferative unresponsiveness and prevents the onset of diabetes in nonobese diabetic mice. J. Exp. Med. 178, 87-99 (1993).
31. Ott, M., Gogvadze, V., Orrenius, S. & Zhivotovsky, B. Mitochondria, oxidative stress and cell death. Apoptosis 12, 913-922 (2007).
32. Seufert, J. et al. Leptin suppression of insulin secretion and gene expression in human pancreatic islets: implications for the development of adipogenic diabetes mellitus. J Clin Endocrinol Metab 84, 670-676 (1999).
33. Kadowaki, T. et al. Adiponectin and adiponectin receptors in insulin resistance, diabetes, and the metabolic syndrome. J. Clin. Invest. 116, 1784-1792 (2006).
34. Truyen, I. et al. Adiponectin levels do not predict clinical onset of type 1 diabetes in antibody-positive relatives. Diabetologia 50, 2143-2146 (2007).
35. Matarese, G., Leiter, E. H. & Cava, A. L. Leptin in autoimmunity: many questions, some answers. Tissue Antigens 70, 87-95 (2007).
36. De Rosa, V. et al. A key role of leptin in the control of regulatory T cell proliferation. Immunity 26, 241-255 (2007).
37. Lord, G. M. et al. Leptin modulates the T-cell immune response and reverses starvation-induced immunosuppression. Nature 394, 897-901 (1998).
38. Ozata, M., Ozdemir, I. C. & Licinio, J. Human leptin deficiency caused by a missense mutation: multiple endocrine defects, decreased sympathetic tone, and immune system dysfunction indicate new targets for leptin action, greater central than peripheral resistance to the effects of leptin, and spontaneous correction of leptin-mediated defects. J. Clin. Endocrinol. Metab. 84, 3686-3695 (1999).
39. Matarese, G. et al. Leptin accelerates autoimmune diabetes in female NOD mice. Diabetes 51, 1356-1361 (2002).
40. Ozcan, L. et al. Endoplasmic reticulum stress plays a central role in development of leptin resistance. Cell Metab. 9, 35-51 (2009).
41. Eizirik, D. L., Colli, M. L. & Ortis, F. The role of inflammation in insulitis and {beta}-cell loss in type 1 diabetes. Nat. Rev. Endocrinol. 5, 219-226 (2009).
42. Hypponen, E. et al. Obesity, increased linear growth, and risk of type 1 diabetes in children. Diabetes Care 23, 1755-1760 (2000).
43. Kimm, S. Y. S. & Obarzanek, E. Childhood obesity: a new pandemic of the new millennium. Pediatrics 110, 1003-1007 (2002).
44. Allison, S. D. & Martiny, J. B. H. Resistance, resilience, and redundancy in microbial communities. Proc. Natl. Acad. Sci. USA 105, 11512-11519 (2008).
45. Turnbaugh, P. J. et al. A core gut microbiome in obese and lean twins. Nature 457, 480-484 (2009).
46. Sokol, H. et al. Faecalibacterium prausnitzii is an anti-inflammatory commensal bacterium identified by gut microbiota analysis of Crohn disease patients. Proc. Natl. Acad. Sci. USA 105, 16731-16736 (2008).
47. Storey, J. D. A direct approach to false discovery rates. J. R. Stat. Soc. B 64, 479-498 (2002).

**References in Examples**

1a. Szot, G. L., Koudria, P. & Bluestone, J. A. Murine pancreatic islet isolation. J. Vis. Exp., 255 (2007).
2a. Ronkainen, M. S. et al. Pregnancy induces nonimmunoglobulin insulin-binding activity in both maternal and cord blood serum. Clin. Exp. Immunol. 124, 190-196 (2001).
3a. Katajamaa, M., Miettinen, J. & Oresic, M. MZmine: toolbox for processing and visualization of mass spectrometry based molecular profile data. Bioinformatics 22, 634-636 (2006).
4a. Katajamaa, M. & Oresic, M. Processing methods for differential analysis of LC/MS profile data. BMC Bioinformatics 6, 179 (2005).
5a. Yetukuri, L. et al. Bioinformatics strategies for lipidomics analysis: characterization of obesity related hepatic steatosis. BMC Syst. Biol. 1, e12 (2007).
6a. Dunning, M. J., Smith, M. L., Ritchie, M. E. & Tavare, S. beadarray: R classes and methods for Illumina bead-based data. Bioinformatics 23, 2183-2184 (2007).
7a. Gentleman, R. et al., Bioinformatics and computational biology solutions using R and Bioconductor. (2005).
8a. Bolstad, B. M., Irizarry, R. A., Astrand, M. & Speed, T. P. A comparison of normalization methods for high density oligonucleotide array data based on variance and bias. Bioinformatics 19, 185-193 (2003).
9a. Subramanian, A. et al. Gene set enrichment analysis: A knowledge-based approach for interpreting genome-wide expression profiles. Proc. Nat. Acad. Sci. U S A 102, 15545-15550 (2005).
10a. Smyth, G. K. Linear models and empirical bayes methods for assessing differential expression in microarray experiments. Stat. Appl. Genet. Mol. Biol. 3, doi: 10.2202/1544-6115.1027 (2004).
11a. Ashburner, M. et al. Gene ontology: tool for the unification of biology. Nat. Genet. 25, 25 - 29 (2000).
12a. Salomonis, N. et al. GenMAPP 2: new features and resources for pathway analysis. BMC Bioinformatics 8, 217 (2007).
13a. Kanehisa, M. et al. KEGG for linking genomes to life and the environment. Nucl. Acids Res. 36, D480-484 (2008).
14a. Jain, A. K., Murty, M. N. & Flynn, P. J. Data clustering: a review. Algorithms for Clustering Data 31, 264-323 (1999).
15a. Maukonen, J. et al. Prevalence and temporal stability of selected clostridial groups in irritable bowel syndrome in relation to predominant faecal bacteria. J Med Microbiol 55, 625-633 (2006).
16a. Maukonen, J., Matto, J., Suihko, M.-L. & Saarela, M. Intra-individual diversity and similarity of salivary and faecal microbiota. J. Med. Microbiol. 57, 1560-1568 (2008).
17a. Storey, J. D. A direct approach to false discovery rates. J. R. Stat. Soc. B 64, 479-498 (2002).
18a. Storey, J. D. & Tibshirani, R. Statistical significance for genomewide studies. Proc. Natl. Acad. Sci. USA 100, 9440-9445 (2003).
19a. Sharma, S., Applied multivariate techniques, Har/Dis edition ed. (Wiley, 1995).
20a. Nikkilä, J. et al. Gender dependent progression of systemic metabolic states in early childhood. Mol. Syst. Biol. 4, e197 (2008).
21a. Tripathi, A., Klami, A. & Kaski, S.,Using dependencies to pair samples for multi-view learning. In TKK Reports in Information and Computer Science, Report No. TKK-ICS-R8 (2008).

**References in Supplementary Figures**

1b. Oresic, M. et al. Dysregulation of lipid and amino acid metabolism precedes islet autoimmunity in children who later progress to type 1 diabetes. J. Exp. Med. 205, 2975 (2008).
2b. Subramanian, A. et al. Gene set enrichment analysis: A knowledge-based approach for interpreting genome-wide expression profiles. Proc. Nat. Acad. Sci. U S A 102, 15545 (2005).
3b. Storey, J. D. A direct approach to false discovery rates. J. R. Stat. Soc. B 64, 479 (2002).
4b. Welthagen, W. et al. Comprehensive two-dimensional gas chromatography time- of flight mass spectrometry (GC-GC-TOF) for high resolution metabolomics: biomarker discovery on spleen tissue extracts of obese NZO compared to lean C57BL/6 mice. Metabolomics 1, 65 (2005).

## Claims

**1.** A method for diagnosing a risk of type 1 diabetes in an individual comprising the steps of

(a) providing a fecal sample from said individual;
(b) analyzing diversity of the *Clostridium leptum* group of said sample, the *Clostridium leptum* group including *Acetanaerobacterium elongatum, Anaerophilum agile, Anaerofilum pentosovorans, Anaerotruncus colihominis, Butyricoccus pullicaecorum, Clostridium cellulosi, Clostridium leptum, Clostridium methylpentosum, Clostridium orbiscindens, Clostridium sporosphaeroides, Clostridium viride, Ethanoligenes harbinense, Eubacterium desmolans, Eubacterium siraeum*, *Eubacterium plautii, Faecalibacterium prausnitzii, Hydrogenoanaerobacterium saccharovorans, Oscillibacter valericigenes, Papillibacter cinnamivorans, Pseudoflavonifractor capillosus, Ruminococcus albus, Ruminococcus bromii, Ruminococcus callidus, Ruminococcus flavefaciens, Sporobacter termitidis*, and *Subdoligranulum variabile;*
(c) comparing the result to control samples obtained from healthy individuals who are not considered at-risk of type 1 diabetes;
(d) determining the difference between said sample and the control samples,
wherein diminished diversity of *Clostridium leptum* group bacteria in said individual as compared to the control samples indicates an increased risk of type 1 diabetes.

**2.** The method of claim 1, wherein the diversity of *Clostridium leptum* group bacteria in a sample is determined as follows:

(a) DNA is extracted from a sample
(b) PCR is performed with primers 5'-GCACAAGCAGTGGAGT-3' and 5'-CGCCCGGGGCGCGCCCCGGGCGGGGCGGGGGCACGGGG GGGTTTTRTCAACGGCAGTC-3'
(c) DGGE analysis is performed using acrylamide-bisacrylamide (37.5:1) gels with linear denaturing gradients
(d) The DGGE gels are analyzed,
wherein diminished diversity of *Clostridium leptum* group bacteria indicates an increased risk of type 1 diabetes.

**3.** The method of claim 1 or 2, wherein genetic background of an individual is estimated.

4. The method of any preceding claims, wherein said individual has an increased risk of type 1 diabetes or is susceptible for developing type 1 diabetes.

## Patentansprüche

1. Ein Verfahren zur Diagnose des Risikos von Typ I Diabetes in einem Individuum, umfassend die Schritte des:

   (a) Bereitstellens einer Stuhlprobe des Individuums;
   (b) Untersuchens der Vielfalt der *Clostridium leptum* Gruppe der Probe, wobei die *Clostridium leptum* Gruppe *Acetanaerobacterium elongatum, Anaerophilum agile, Anaerofilum pentosovorans, Anaerotruncus colihominis, Butyricoccus pllicaecorum, Clostridium cellulosi, Clostridium leptum, Clostridium methylpentosum, Clostridium orbiscindens, Clostridium sporosphaeroides, Clostridium viride, Ethanoligenes harbinense, Eubacterium desmolans, Eubacterium siraeum, Eubacterium plautii, Faecalibacterium prausnitzii, Hydrogenoanaerobacterium saccharovorans, Oscillibacter valericigenes, Papillibacter cinnamivorans, Pseudoflavonifractor capillosus, Ruminococcus albus, Ruminococcus bromii, Ruminococcus callidus, Ruminococcus flavefaciens, Sporobacter termitidis,* und *Subdoligranulum variabile* einschließt;
   (c) Vergleichens des Ergebnisses mit Kontrollproben, die von gesunden Individuen erhalten wurden, von denen angenommen wird, dass sie kein Risiko für Typ I Diabetes haben;
   (d) Bestimmens des Unterschieds zwischen der Probe und den Kontrollproben, wobei eine im Vergleich mit den Kontrollproben verminderte Vielfalt von Bakterien der *Clostridium leptum* Gruppe in dem Individuum ein Risiko für Typ I Diabetes anzeigt.

2. Das Verfahren gemäß Anspruch 1, wobei die Vielfalt der Bakterien der *Clostridium leptum* Gruppe in einer Probe wie folgt bestimmt wird:

   (a) DNS wird aus einer Probe extrahiert
   (b) eine PCR wird mit den Primern 5 '-GCACAAGCAGTGGAGT-3 ' und 5 '-CGCCCGGGGCGCGCCCCGGG-CGGGGCGGGGGCACGGGGGGTTTTR TCAACGGCAGTC-3' durchgeführt.
   (c) eine DGGE-Analyse wird unter Verwendung von Acrylamid-Bisacrylamid-(37.5:1) Gelen mit linearen Denaturierungsgradienten durchgeführt
   (d) die DGGE-Gele werden untersucht,
   wobei eine verminderte Vielfalt von Bakterien der *Clostridium leptum* Gruppe ein Risiko für Typ I Diabetes anzeigt.

3. Das Verfahren gemäß Anspruch 1 oder 2, wobei der genetische Hintergrund des Individuums geschätzt wird.

4. Das Verfahren gemäß einem der vorangehenden Ansprüche, wobei das Individuum ein erhöhtes Risiko für Typ I Diabetes aufweist oder anfällig dafür ist, einen Typ I Diabetes zu entwickeln.

## Revendications

1. Méthode destinée à diagnostiquer un risque de diabète de type 1 chez un individu comprenant les étapes suivantes :

   (a) la fourniture d'un échantillon fécal provenant dudit individu ;
   (b) l'analyse de la diversité des bactéries du groupe *Clostridium leptum* dans ledit échantillon, le groupe *Clostridium leptum* comprenant : *Acetanaerobacterium elongatum, Anaerophilum agile, Anaerofilum pentosovorans, Anaerotruncus colihominis, Butyricoccus pullicaecorum, Clostridium cellulosi, Clostridium leptum, Clostridium methylpentosum, Clostridium orbiscindens, Clostridium sporosphaeroides, Clostridium viride, Ethanoligenes harbinense, Eubacterium desmolans, Eubacterium siraeum, Eubacterium plautii, Faecalibacterium prausnitzii, Hydrogenoanaerobacterium saccharovorans, Oscillibacter valericigenes, Papillibacter cinnamivorans, Pseudoflavonifractor capillosus, Ruminococcus albus, Ruminococcus bromii, Ruminococcus callidus, Ruminococcus flavefaciens, Sporobacter termitidis,* et *Subdoligranulum variabile ;*
   (c) la comparaison du résultat à des échantillons témoins provenant d'individus sains qui ne sont pas considérés comme à risque de développer un diabète de type 1 ;
   (d) la détermination de la différence entre ledit échantillon et les échantillons témoins, dans laquelle une diversité réduite des bactéries du groupe *Clostridium leptum* chez ledit individu indique un risque accru de développer un diabète de type 1.

**2.** Méthode selon la revendication 1, dans laquelle la diversité des bactéries du groupe *Clostridium leptum* dans un échantillon est déterminée comme suit :

(a) l'ADN est extrait de l'échantillon
(b) une PCR est mise en oeuvre avec les amorces 5'-GCACAAGCAGTGGAGT-3' et 5'-CGCCCGGGGCGCGCCCCGGGCGGGGCGGGGGCACGGGG GGGTTTTRTCAACGGCAGTC-3 '
(c) une analyse DGGE est mise en oeuvre à l'aide de gels d'acrylamide-bisacrylamide (37,5:1) ayant des gradients de dénaturation linéaires
(d) les gels DGGE sont analysés,
une diversité réduite des bactéries du groupe *Clostridium leptum* indiquant un risque accru de développer un diabète de type 1.

**3.** Méthode selon la revendication 1 ou 2, dans laquelle le contexte génétique de l'individu est estimé.

**4.** Méthode selon l'une quelconque des revendications précédentes, dans laquelle ledit individu présente un risque accru de diabète de type 1 et est susceptible de développer un diabète de type 1.

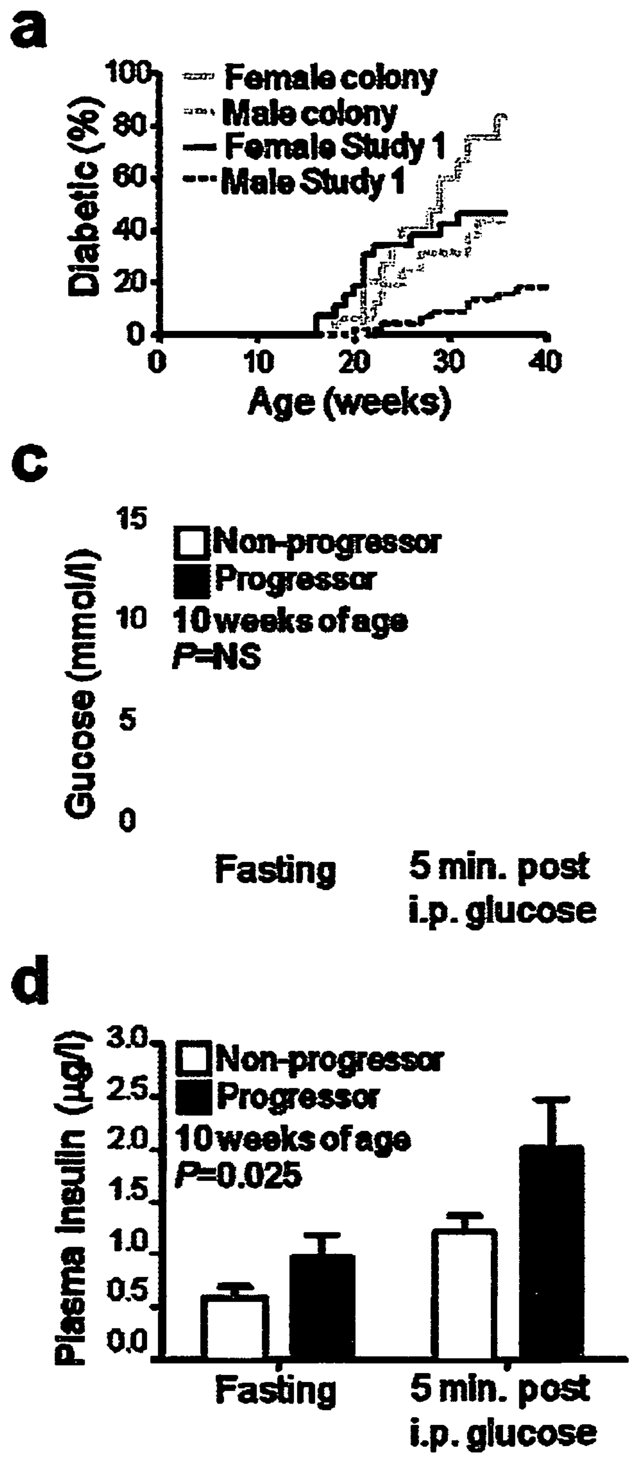
a
100
80
60
40
20
0
0
10
20
30
40
Diabetic (%)
Age (weeks)
Female colony
Male colony
Female Study 1
Male Study 1
c
15
10
5
0
Gucose (mmol/l)
Non-progressor
Progressor
10 weeks of age
P=NS
Fasting
5 min. post
i.p. glucose
d
3.0
2.5
2.0
1.5
1.0
0.5
0.0
Plasma Insulin (μg/l)
Non-progressor
Progressor
10 weeks of age
P=0.025
Fasting
5 min. post
i.p. glucose

Fig. 1

b

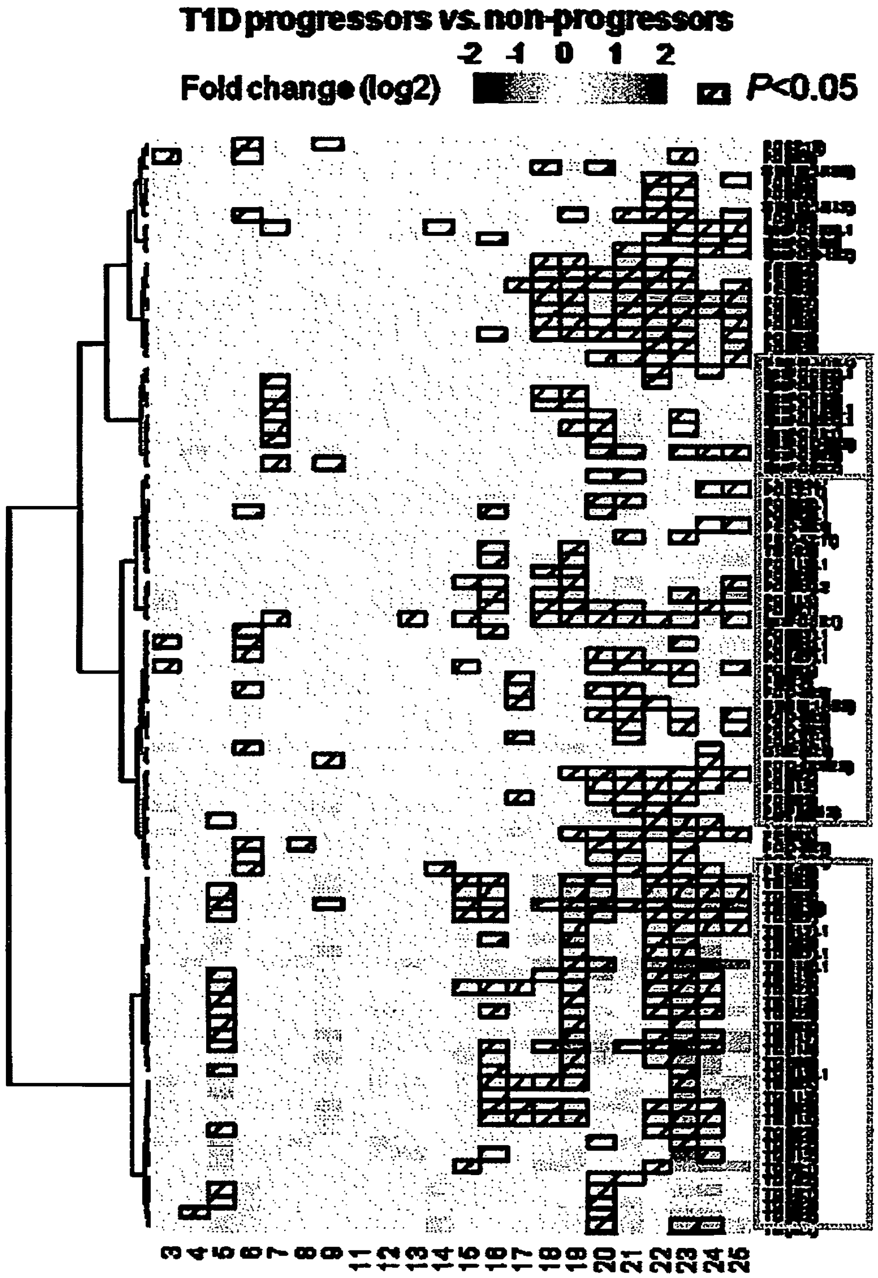
T1D progressors vs. non-progressors
-2 -1 0 1 2
Fold change (log2)
P<0.05
Mostly lysoPC
Mostly PC
Mostly triacylglycerols
3 4 5 6 7 8 9 10 11 12 13 14 15 16 17 18 19 20 21 22 23 24 25

Fig. 1 cont.

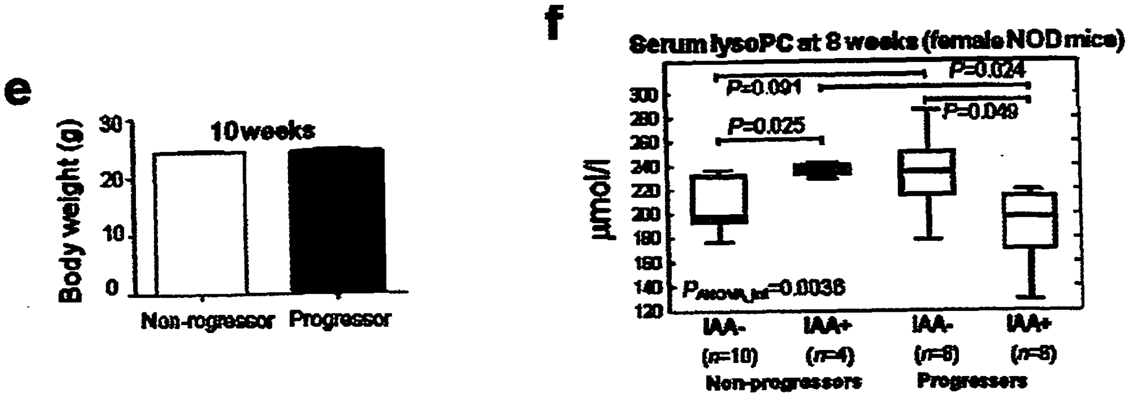
e
10weeks
Body weight (g)
30
20
10
0
Non-rogressor
Progressor
f
Serum lysoPC at 8 weeks (female NOD mice)
P=0.091
P=0.024
P=0.049
P=0.025
μmol/l
300
280
260
240
220
200
180
160
140
120
IAA-
(n=10)
IAA+
(n=4)
Non-progressors
IAA-
(n=8)
IAA+
(n=8)
Progressors

Fig. 1 cont.

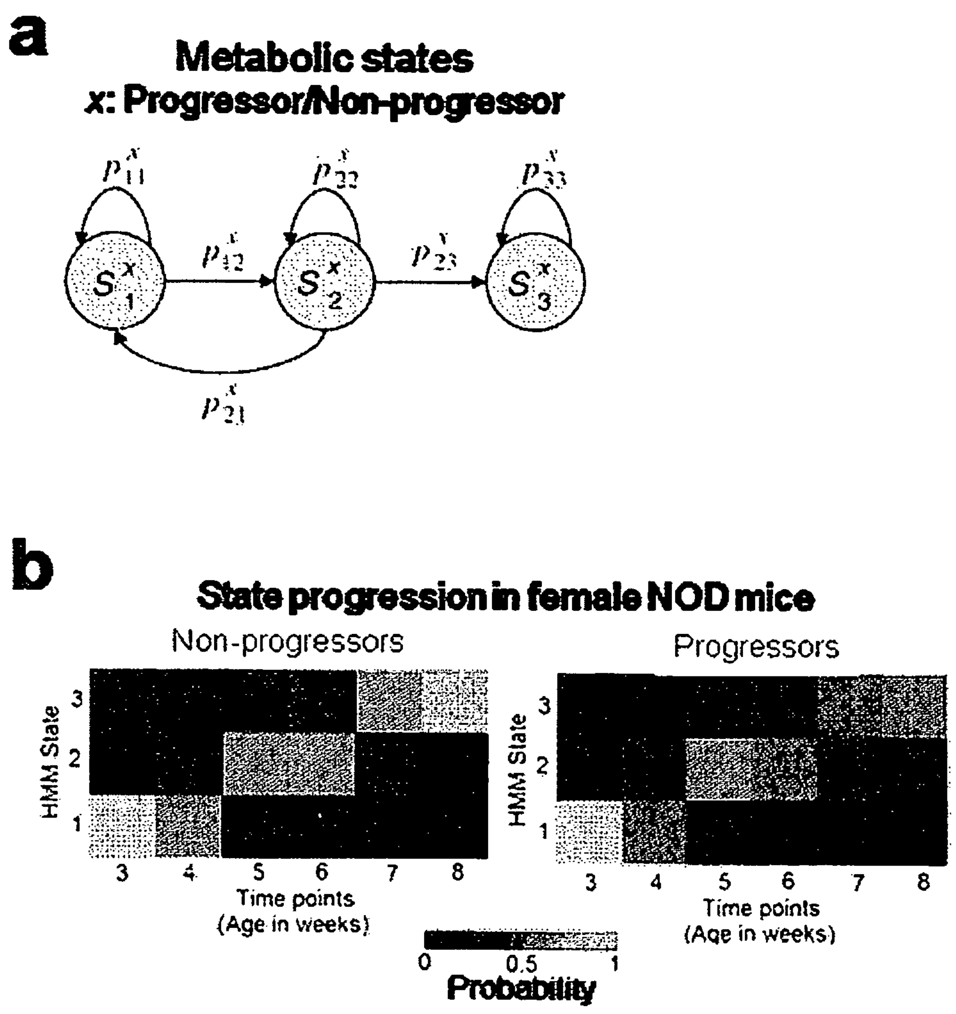
a
Metabolic states
x: Progressor/Non-progressor
b
State progression in female NOD mice
Non-progressors
Progressors
HMM State
Time points
(Age in weeks)
Probability

Fig. 2

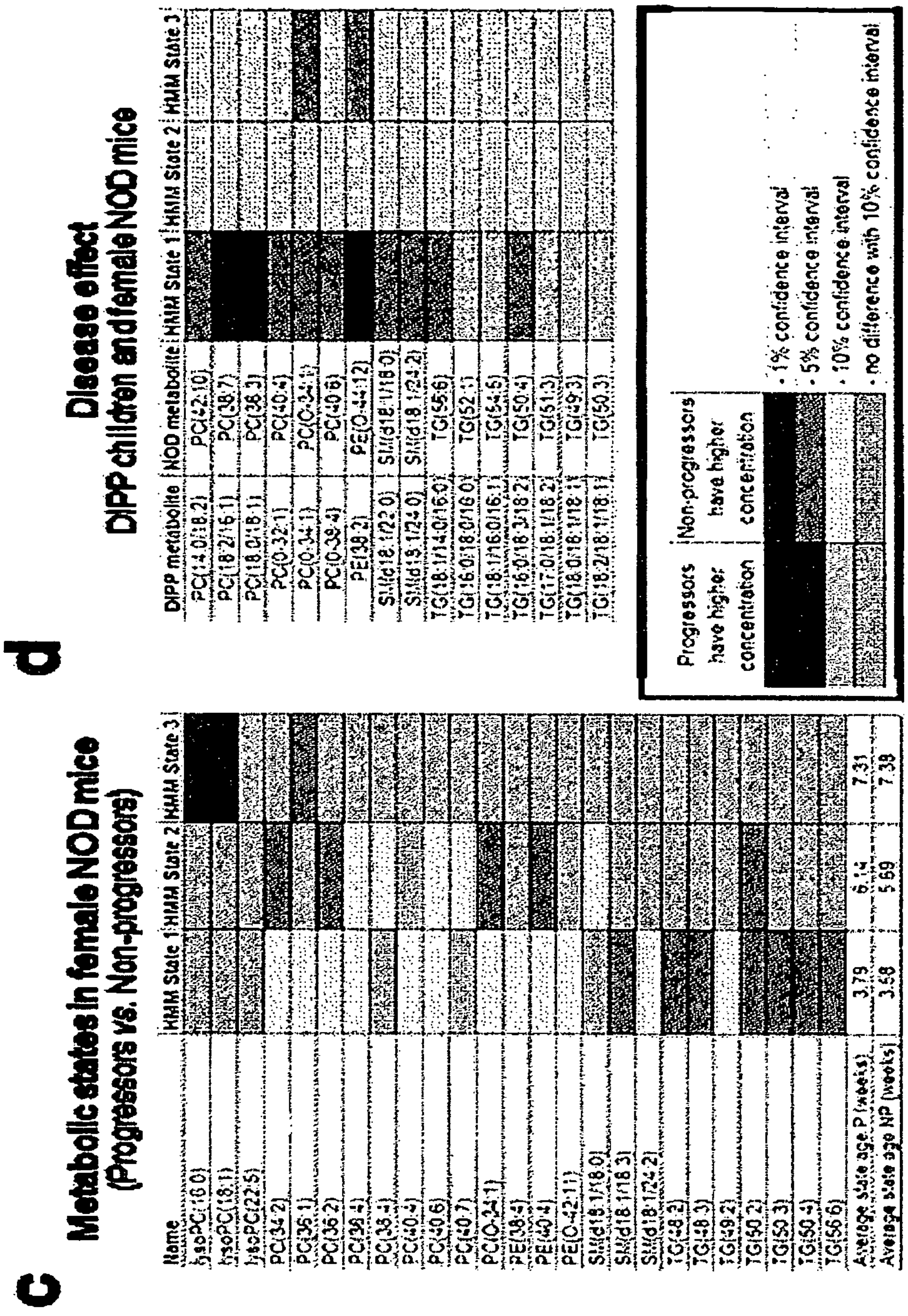
c
Metabolic states in female NOD mice
(Progressors vs. Non-progressors)
Name
HMM State 1
HMM State 2
HMM State 3
Average state age P (weeks)
Average state age NP (weeks)
3.79
3.58
6.14
5.69
7.31
7.38
d
Disease effect
DIPP children and female NOD mice
DIPP metabolite
NOD metabolite
Progressors have higher concentration
Non-progressors have higher concentration
1% confidence interval
5% confidence interval
10% confidence interval
no difference with 10% confidence interval

Fig. 2 cont.

a

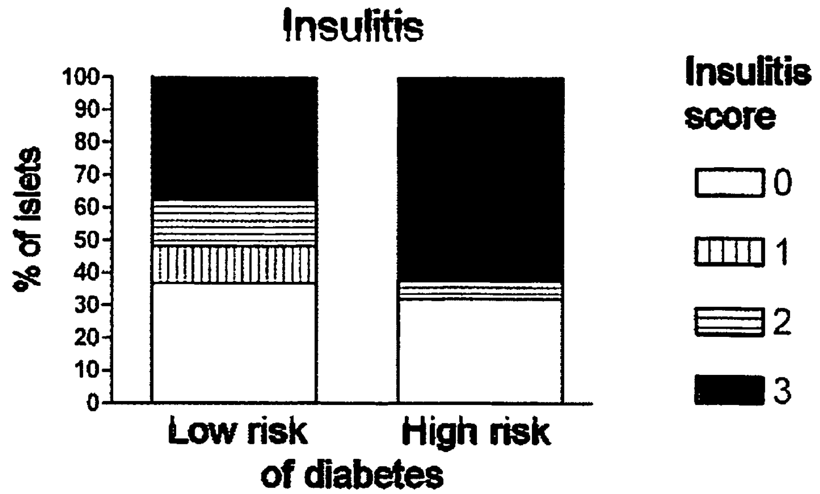
Insulitis
100
90
80
70
60
50
40
30
20
10
0
% of Islets
Low risk
High risk
of diabetes
Insulitis score
0
1
2
3

c

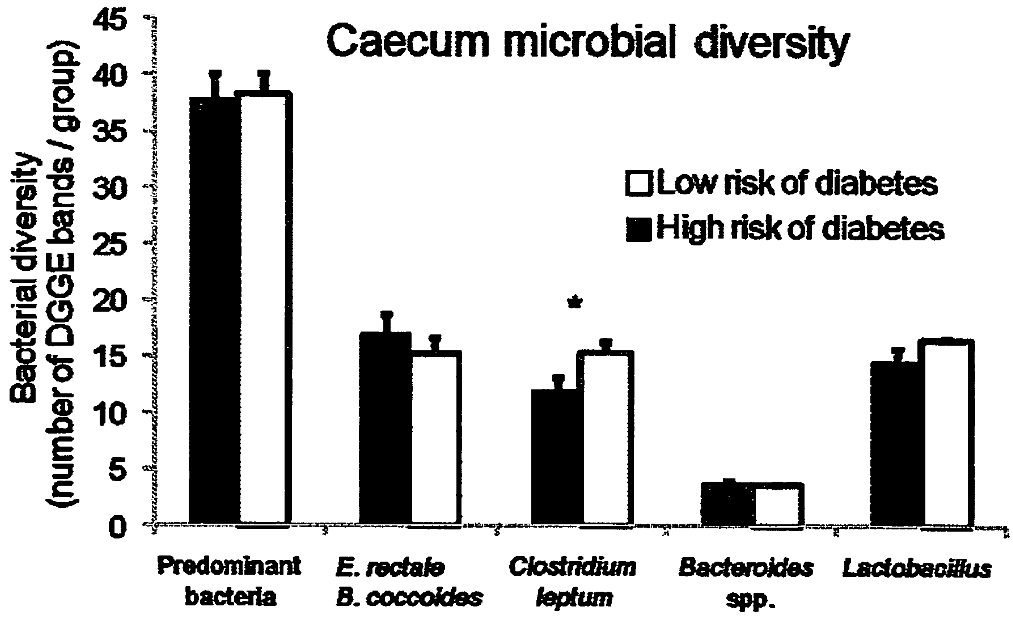
Caecum microbial diversity
Bacterial diversity
(number of DGGE bands / group)
45
40
35
30
25
20
15
10
5
0
Low risk of diabetes
High risk of diabetes
*
Predominant bacteria
E. rectale B. coccoides
Clostridium leptum
Bacteroides spp.
Lactobacillus

Fig. 3

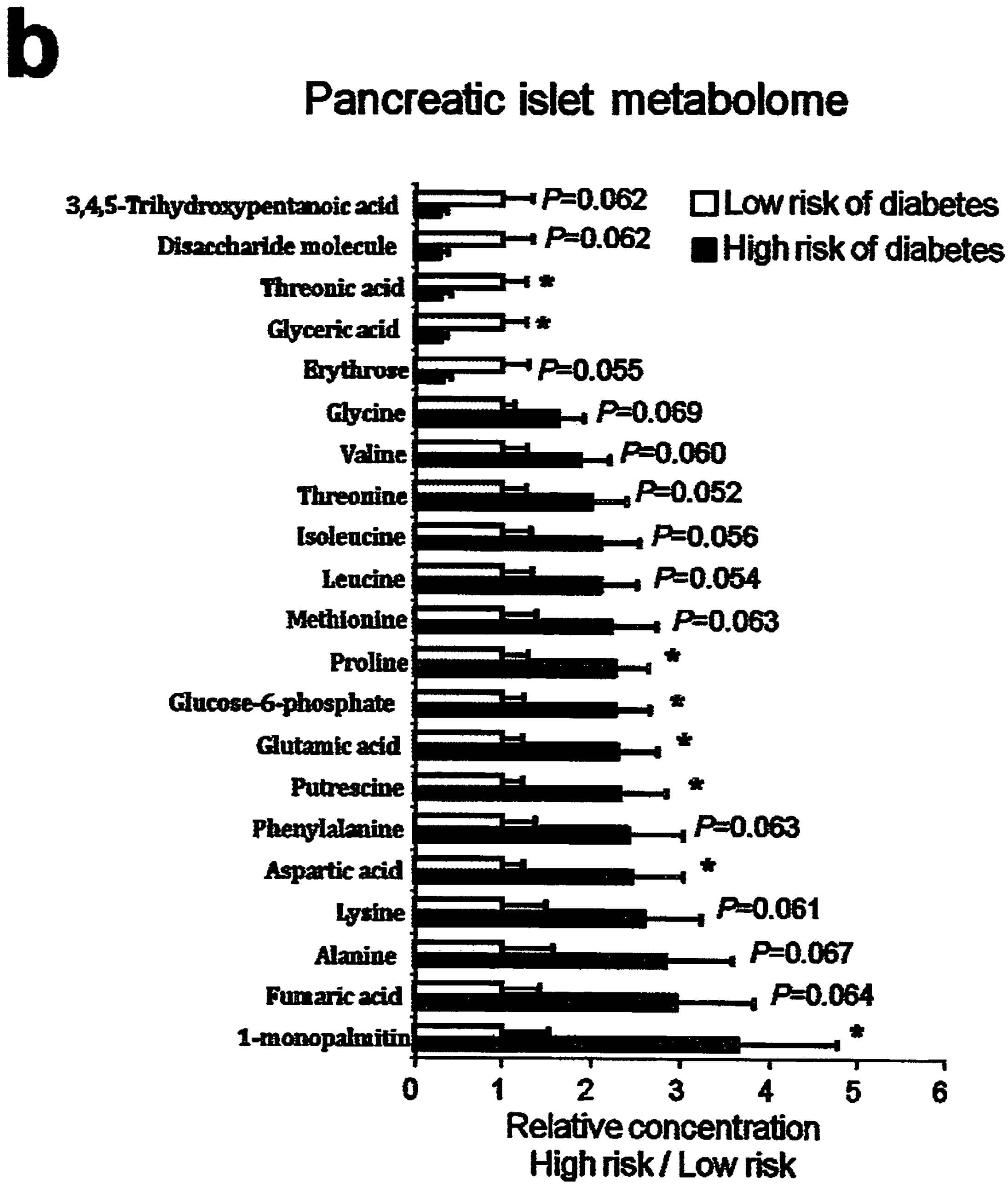
b
Pancreatic islet metabolome
3,4,5-Trihydroxypentanoic acid
Disaccharide molecule
Threonic acid
Glyceric acid
Erythrose
Glycine
Valine
Threonine
Isoleucine
Leucine
Methionine
Proline
Glucose-6-phosphate
Glutamic acid
Putrescine
Phenylalanine
Aspartic acid
Lysine
Alanine
Fumaric acid
1-monopalmitin
P=0.062
P=0.062
*
*
P=0.055
P=0.069
P=0.060
P=0.052
P=0.056
P=0.054
P=0.063
*
*
*
*
P=0.063
*
P=0.061
P=0.067
P=0.064
*
Low risk of diabetes
High risk of diabetes
0
1
2
3
4
5
6
Relative concentration
High risk / Low risk

Fig. 3 cont.

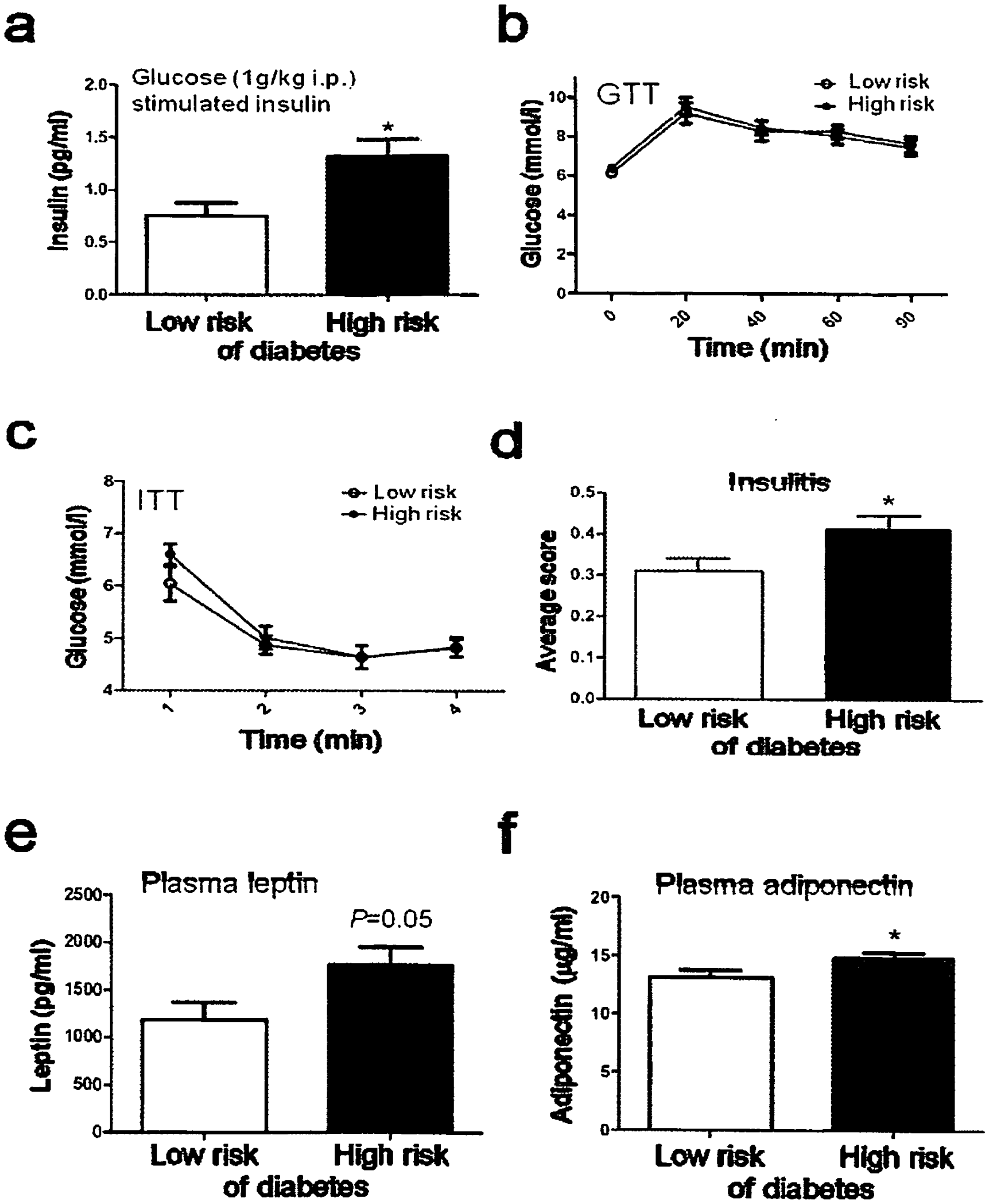
a
Glucose (1g/kg i.p.) stimulated insulin
Insulin (pg/ml)
2.0
1.5
1.0
0.5
0.0
*
Low risk
High risk
of diabetes
b
GTT
Low risk
High risk
Glucose (mmol/l)
10
8
6
4
2
0
0
20
40
60
90
Time (min)
c
ITT
Low risk
High risk
Glucose (mmol/l)
8
7
6
5
4
1
2
3
4
Time (min)
d
Insulitis
Average score
0.5
0.4
0.3
0.2
0.1
0.0
*
Low risk
High risk
of diabetes
e
Plasma leptin
P=0.05
Leptin (pg/ml)
2500
2000
1500
1000
500
0
Low risk
High risk
of diabetes
f
Plasma adiponectin
Adiponectin (µg/ml)
20
15
10
5
0
*
Low risk
High risk
of diabetes

Fig. 4

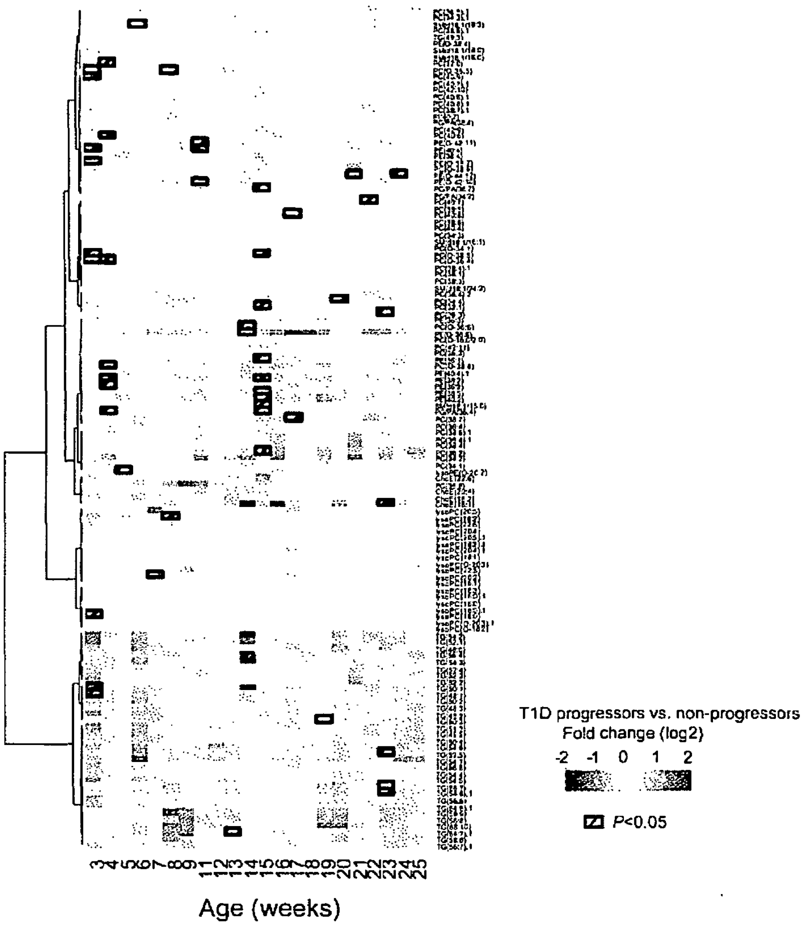


**Supplementary Fig. 1**

**a**

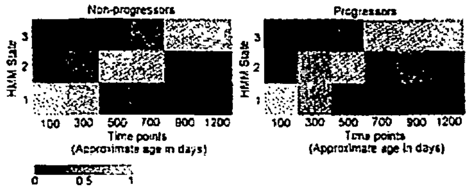


**b**

| Name | HMM State 1 | HMM State 2 | HMM State 3 |
|---|---|---|---|
| Cer(d18:1/22:0) | | | |
| lysoPC(18:2) | | | |
| PC(14:0/18:2) | | | |
| PC(18:0/18:1) | | | |
| PC(18:2/16:1) | | | |
| PC(18:2/18:2) | | | |
| PC(38:7) | | | |
| PC(O-16:0/20:4) | | | |
| PC(O-18:1/20:4) | | | |
| PC(O-32:1) | | | |
| PC(O-34:1) | | | |
| PC(O-38:4) | | | |
| PE(38:1) | | | |
| PE(38:2) | | | |
| SM(d18:1/16:1) | | | |
| SM(d18:1/22:0) | | | |
| SM(d18:1/23:1) | | | |
| SM(d18:1/24:0) | | | |
| SM(d18:1/25:1) | | | |
| TG(16:0/14:0/16:0) | | | |
| TG(16:0/16:0/16:0) | | | |
| TG(16:0/18:0/18:0) | | | |
| TG(16:0/18:1/15:0) | | | |
| TG(16:0/18:1/17:0) | | | |
| TG(16:0/18:1/18:0) | | | |
| TG(16:0/18:2/14:1) | | | |
| TG(16:0/18:2/18:2) | | | |
| TG(16:0/18:3/18:2) | | | |
| TG(16:1/18:1/18:1) | | | |
| TG(17:0/18:1/18:2) | | | |
| TG(18:0/18:1/18:1) | | | |
| TG(18:1/14:0/16:0) | | | |
| TG(18:1/16:0/12:0) | | | |
| TG(18:1/18:1/15:0) | | | |
| TG(18:1/18:1/18:1) | | | |
| TG(18:1/18:2/18:2) | | | |
| TG(18:2/18:1/18:1) | | | |
| TG(42:0) | | | |
| TG(48:2) | | | |
| Average age P (days) | 319 | 662 | 1181 |
| Average age NP (days) | 277 | 731 | 1146 |

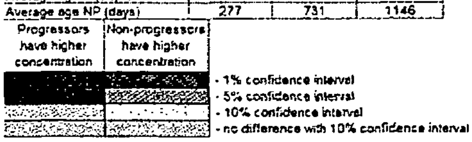


**Supplementary Fig. 2**

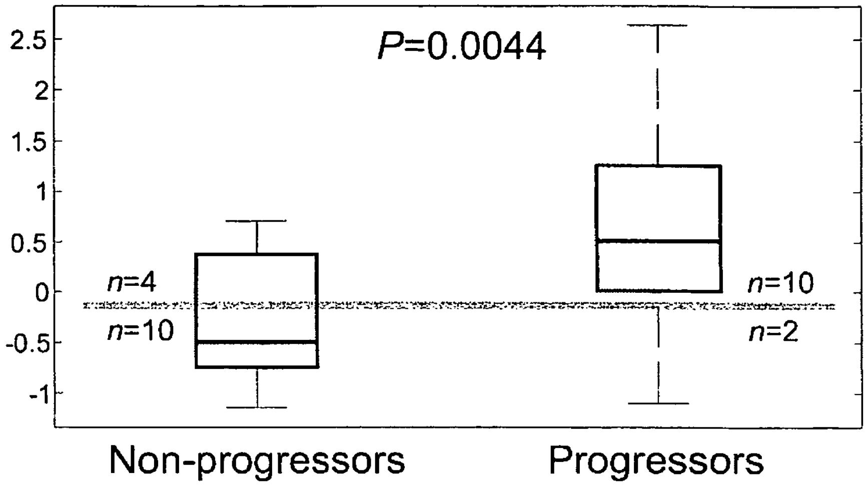


**Supplementary Fig. 3**

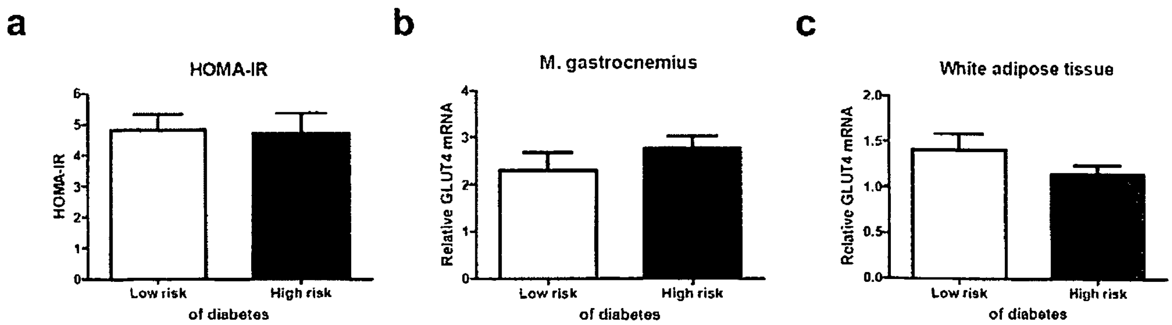


**Supplementary Fig. 4**

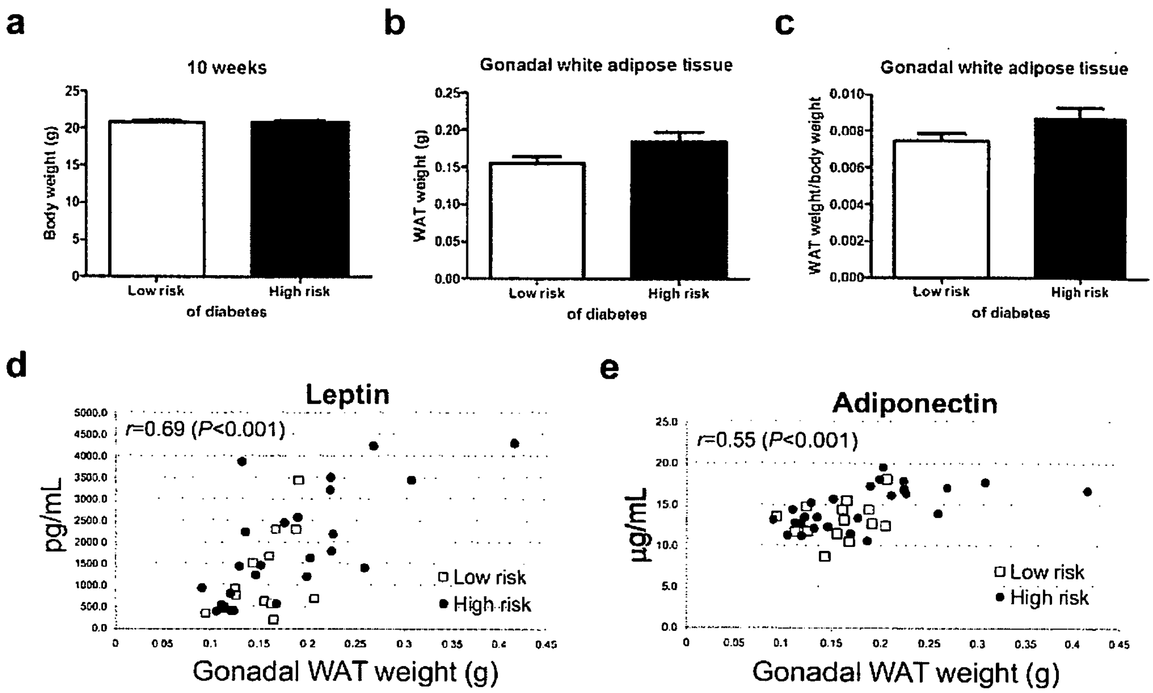


# Supplementary Figure 5.

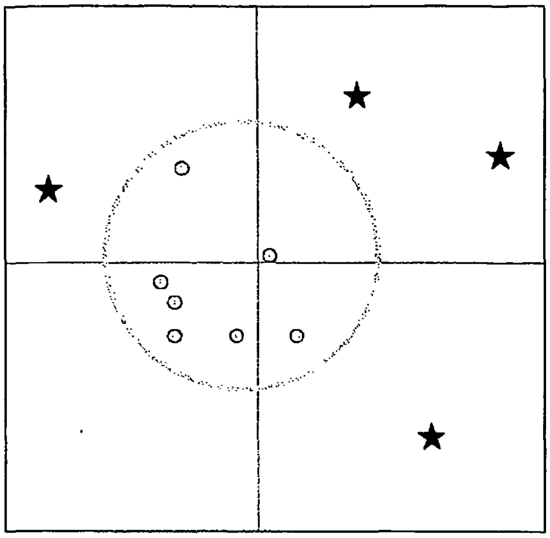

Supplementary Fig. 6

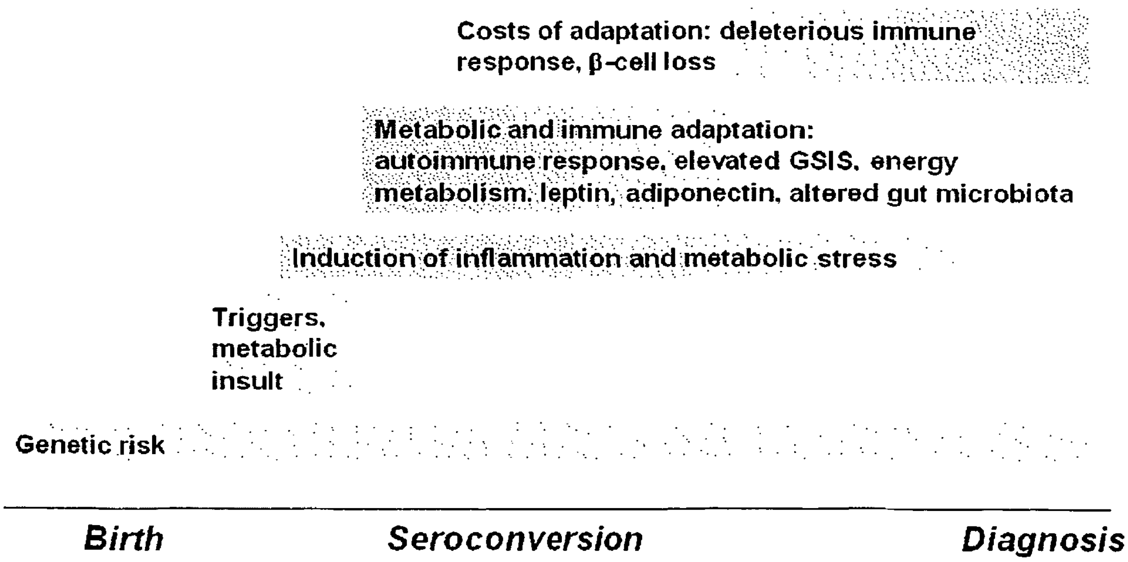


Supplementary Fig. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008031917 A


### Non-patent literature cited in the description


- **LUIZ FW ROESCH et al.** teaches identification of bacteria (in particular Lactobacillus and Bacteroides strais) correlated with the onset of diabetes in a rat model. *THE I S M E JOURNAL,* 01 May 2009, vol. 3 (5), 536-548
- **ACHENBACH, P. ; BONIFACIO, E. ; KOCZWARA, K. ; ZIEGLER, A.-G.** Natural history of type 1 diabetes. *Diabetes,* 2005, vol. 54, 25-31
- **ORESIC, M. et al.** Dysregulation of lipid and amino acid metabolism precedes islet autoimmunity in children who later progress to type 1 diabetes. *J. Exp. Med.,* 2008, vol. 205, 2975-2984
- **GALE, E. A. M.** The rise of childhood type 1 diabetes in the 20th century. *Diabetes,* 2002, vol. 51, 3353-3361
- **HARJUTSALO, V. ; SJÖBERG, L. ; TUOMILEHTO, J.** Time trends in the incidence of type 1 diabetes in Finnish children: a cohort study. *Lancet,* 2008, vol. 371, 1777-1782
- **PATTERSON, C. C. et al.** Incidence trends for childhood type 1 diabetes in Europe during 1989-2003 and predicted new cases 2005-20: a multicentre prospective registration study. *Lancet,* 2009, vol. 373, 2027-2033
- **ORESIC, M. ; VIDAL-PUIG, A. ; HÄNNINEN, V.** Metabolomic approaches to phenotype characterization and applications to complex diseases. *Expert Rev. Mol. Diagn.,* 2006, vol. 6, 575-585
- **HAGOPIAN, W. A. et al.** TEDDY - the environmental determinants of diabetes in the young: an observational clinical trial. *Ann. NY Acad. Sci.,* 2006, vol. 1079, 320-326
- **KUPILA, A. et al.** Feasibility of genetic and immunological prediction of type 1 diabetes in a population-based birth cohort. *Diabetologia,* 2001, vol. 44, 290-297
- **ANDERSON, M. S. ; BLUESTONE, J. A.** The NOD mouse: a model of immune dysregulation. *Annu. Rev. Immunol.,* 2005, vol. 23, 447-485
- **ATKINSON, M. A. ; LEITER, E. H.** The NOD mouse model of type 1 diabetes: as good as it gets?. *Nature,* 1999, vol. 5, 601-604
- **CHERVONSKY, A. V.** Influence of microbial environment on autoimmunity. *Nat. Immunol.,* 2010, vol. 11, 28-35
- **SINGH, B. ; RABINOVITCH, A.** Influence of microbial agents on the development and prevention of autoimmune diabetes. *Autoimmunity,* 1993, vol. 15, 209-213
- **WEN, L. et al.** Innate immunity and intestinal microbiota in the development of type 1 diabetes. *Nature,* 2008, vol. 455, 1109-1113
- **FLOYD, J. C. J. ; FAJANS, S. S. ; KNOPF, R. F. ; CONN, J. W.** Evidence that insulin release is the mechanism for experimentally induced leucine hypoglycemia in man. *J. Clin. Invest.,* 1963, vol. 42, 1714-1719
- **NIKKILÄ, J. et al.** Gender dependent progression of systemic metabolic states in early childhood. *Mol. Syst. Biol.,* 2008, vol. 4, 197
- **TRIPATHI, A. ; KLAMI, A. ; KASKI, S.** Using dependencies to pair samples for multi-view learning. *TKK Reports in Information and Computer Science, Report No. TKK-ICS-R8,* 2008
- **SUBRAMANIAN, A. et al.** Gene set enrichment analysis: A knowledge-based approach for interpreting genome-wide expression profiles. *Proc. Nat. Acad. Sci. U S A,* 2005, vol. 102, 15545-15550
- **KEYMEULEN, B. et al.** Insulin needs after CD3-Antibody therapy in new-onset type 1 diabetes. *N. Engl. J. Med.,* 2005, vol. 352, 2598-2608
- **EIZIRIK, D. L. ; MOORE, F. ; FLAMEZ, D. ; ORTIS, F.** Use of systems biology approach to understand pancreatic {beta}-cell death in type 1 diabetes. *Biochem. Soc. Trans.,* 2008, vol. 36, 321-327
- **ZHERNAKOVA, A. et al.** Genetic variants of RANTES are associated with serum RANTES level and protection for type 1 diabetes. *Genes Immun.,* 2006, vol. 7, 544-549
- **CALZASCIA, T. et al.** CD4 T cells, lymphopenia, and IL-7 in a multistep pathway to autoimmunity. *Proc. Natl. Acad. Sci. USA,* 2008, vol. 105, 2999-3004

- **WELTHAGEN, W. et al.** Comprehensive two-dimensional gas chromatography time-of-flight mass spectrometry (GC-GC-TOF) for high resolution metabolomics: biomarker discovery on spleen tissue extracts of obese NZO compared to lean C57BL/6 mice. *Metabolomics,* 2005, vol. 1, 65-73
- **NEWSHOLME, P. ; BRENNAN, L. ; BENDER, K.** Amino acid metabolism, {beta}-cell function, and diabetes. *Diabetes,* 2006, vol. 55, 39-47
- **FOURLANOS, S. et al.** Insulin resistance is a risk factor for progression to Type 1 diabetes. *Diabetologia,* 2004, vol. 47, 1661-1667
- **XU, P. et al.** Role of insulin resistance in predicting progression to type 1 diabetes. *Diabetes Care,* 2007, vol. 30, 2314-2320
- **CHAPARRO, R. J. et al.** Nonobese diabetic mice express aspects of both type 1 and type 2 diabetes. *Proc. Natl. Acad. Sci. USA,* 2006, vol. 103, 12475-12480
- **VELAGAPUDI, V. R. et al.** The gut microbiota modulates host energy and lipid metabolism in mice. *J. Lipid Res.,* 2010, vol. 51, 1101-1112
- **MAUKONEN, J. et al.** Prevalence and temporal stability of selected clostridial groups in irritable bowel syndrome in relation to predominant faecal bacteria. *J Med Microbiol,* 2006, vol. 55, 625-633
- **MAUKONEN, J. ; MATTO, J. ; SUIHKO, M.-L. ; SAARELA, M.** Intra-individual diversity and similarity of salivary and faecal microbiota. *J. Med. Microbiol.,* 2008, vol. 57, 1560-1568
- **RAPOPORT, M. et al.** Interleukin 4 reverses T cell proliferative unresponsiveness and prevents the onset of diabetes in nonobese diabetic mice. *J. Exp. Med.,* 1993, vol. 178, 87-99
- **OTT, M. ; GOGVADZE, V. ; ORRENIUS, S. ; ZHIVOTOVSKY, B.** Mitochondria, oxidative stress and cell death. *Apoptosis,* 2007, vol. 12, 913-922
- **SEUFERT, J. et al.** Leptin suppression of insulin secretion and gene expression in human pancreatic islets: implications for the development of adipogenic diabetes mellitus. *J Clin Endocrinol Metab,* 1999, vol. 84, 670-676
- **KADOWAKI, T. et al.** Adiponectin and adiponectin receptors in insulin resistance, diabetes, and the metabolic syndrome. *J. Clin. Invest.,* 2006, vol. 116, 1784-1792
- **TRUYEN, I. et al.** Adiponectin levels do not predict clinical onset of type 1 diabetes in antibody-positive relatives. *Diabetologia,* 2007, vol. 50, 2143-2146
- **MATARESE, G. ; LEITER, E. H. ; CAVA, A. L.** Leptin in autoimmunity: many questions, some answers. *Tissue Antigens,* 2007, vol. 70, 87-95
- **DE ROSA, V. et al.** A key role of leptin in the control of regulatory T cell proliferation. *Immunity,* 2007, vol. 26, 241-255
- **LORD, G. M. et al.** Leptin modulates the T-cell immune response and reverses starvation-induced immunosuppression. *Nature,* 1998, vol. 394, 897-901
- **OZATA, M. ; OZDEMIR, I. C. ; LICINIO, J.** Human leptin deficiency caused by a missense mutation: multiple endocrine defects, decreased sympathetic tone, and immune system dysfunction indicate new targets for leptin action, greater central than peripheral resistance to the effects of leptin, and spontaneous correction of leptin-mediated defects. *J. Clin. Endocrinol. Metab.,* 1999, vol. 84, 3686-3695
- **MATARESE, G. et al.** Leptin accelerates autoimmune diabetes in female NOD mice. *Diabetes,* 2002, vol. 51, 1356-1361
- **OZCAN, L. et al.** Endoplasmic reticulum stress plays a central role in development of leptin resistance. *Cell Metab.,* 2009, vol. 9, 35-51
- **EIZIRIK, D. L. ; COLLI, M. L. ; ORTIS, F.** The role of inflammation in insulitis and {beta}-cell loss in type 1 diabetes. *Nat. Rev. Endocrinol.,* 2009, vol. 5, 219-226
- **HYPPONEN, E. et al.** Obesity, increased linear growth, and risk of type 1 diabetes in children. *Diabetes Care,* 2000, vol. 23, 1755-1760
- **KIMM, S. Y. S. ; OBARZANEK, E.** Childhood obesity: a new pandemic of the new millennium. *Pediatrics,* 2002, vol. 110, 1003-1007
- **ALLISON, S. D. ; MARTINY, J. B. H.** Resistance, resilience, and redundancy in microbial communities. *Proc. Natl. Acad. Sci. USA,* 2008, vol. 105, 11512-11519
- **TURNBAUGH, P. J. et al.** A core gut microbiome in obese and lean twins. *Nature,* 2009, vol. 457, 480-484
- **SOKOL, H. et al.** Faecalibacterium prausnitzii is an anti-inflammatory commensal bacterium identified by gut microbiota analysis of Crohn disease patients. *Proc. Natl. Acad. Sci. USA,* 2008, vol. 105, 16731-16736
- **STOREY, J. D.** A direct approach to false discovery rates. *J. R. Stat. Soc. B,* 2002, vol. 64, 479-498
- **SZOT, G. L. ; KOUDRIA, P. ; BLUESTONE, J. A.** Murine pancreatic islet isolation. *J. Vis. Exp.,* 2007, 255
- **RONKAINEN, M. S. et al.** Pregnancy induces nonimmunoglobulin insulin-binding activity in both maternal and cord blood serum. *Clin. Exp. Immunol.,* 2001, vol. 124, 190-196
- **KATAJAMAA, M. ; MIETTINEN, J. ; ORESIC, M.** MZmine: toolbox for processing and visualization of mass spectrometry based molecular profile data. *Bioinformatics,* 2006, vol. 22, 634-636
- **KATAJAMAA, M. ; ORESIC, M.** Processing methods for differential analysis of LC/MS profile data. *BMC Bioinformatics,* 2005, vol. 6, 179

- **YETUKURI, L. et al.** Bioinformatics strategies for lipidomics analysis: characterization of obesity related hepatic steatosis. *BMC Syst. Biol.,* 2007, vol. 1, 12
- **DUNNING, M. J. ; SMITH, M. L. ; RITCHIE, M. E. ; TAVARE, S.** beadarray: R classes and methods for Illumina bead-based data. *Bioinformatics,* 2007, vol. 23, 2183-2184
- **GENTLEMAN, R. et al.** *Bioinformatics and computational biology solutions using R and Bioconductor.,* 2005
- **BOLSTAD, B. M. ; IRIZARRY, R. A. ; ASTRAND, M. ; SPEED, T. P.** A comparison of normalization methods for high density oligonucleotide array data based on variance and bias. *Bioinformatics,* 2003, vol. 19, 185-193
- **SMYTH, G. K.** Linear models and empirical bayes methods for assessing differential expression in microarray experiments. *Stat. Appl. Genet. Mol. Biol.,* 2004, 3
- **ASHBURNER, M. et al.** Gene ontology: tool for the unification of biology. *Nat. Genet.,* 2000, vol. 25, 25-29
- **SALOMONIS, N. et al.** GenMAPP 2: new features and resources for pathway analysis. *BMC Bioinformatics,* 2007, vol. 8, 217
- **KANEHISA, M. et al.** KEGG for linking genomes to life and the environment. *Nucl. Acids Res.,* 2008, vol. 36, D480-484
- **JAIN, A. K. ; MURTY, M. N. ; FLYNN, P. J.** Data clustering: a review. *Algorithms for Clustering Data,* 1999, vol. 31, 264-323
- **STOREY, J. D. ; TIBSHIRANI, R.** Statistical significance for genomewide studies. *Proc. Natl. Acad. Sci. USA,* 2003, vol. 100, 9440-9445
- **SHARMA, S.** Applied multivariate techniques. Wiley, 1995
- **SUBRAMANIAN, A. et al.** Gene set enrichment analysis: A knowledge-based approach for interpreting genome-wide expression profiles. *Proc. Nat. Acad. Sci. U S A,* 2005, vol. 102, 15545
- **STOREY, J. D.** A direct approach to false discovery rates. *J. R. Stat. Soc. B,* 2002, vol. 64, 479
- **WELTHAGEN, W. et al.** Comprehensive two-dimensional gas chromatography time- of flight mass spectrometry (GC-GC-TOF) for high resolution metabolomics: biomarker discovery on spleen tissue extracts of obese NZO compared to lean C57BL/6 mice. *Metabolomics,* 2005, vol. 1, 65

SEQUENCE LISTING

<110> TEKNOLOGIAN TUTKIMUSKESKUS VTT

<120> METHOD FOR DIAGNOSING RISK OF TYPE 1 DIABETES AND FOR PREVENTING ONSET OF TYPE 1 DIABETES

<130> VTT285PCT

<160> 2

<170> PatentIn version 3.4

<210> 1
<211> 16
<212> DNA
<213> Artificial

<220>
<223> primer

<400> 1
gcacaagcag tggagt 16

<210> 2
<211> 58
<212> DNA
<213> Artificial

<220>
<223> primer

<400> 2
cgcccggggc gcgccccggg cggggcgggg gcacgggggg gttttrtcaa cggcagtc 58